# EUROPEAN PATENT APPLICATION

(11) **EP 4 019 537 A1**
(43) Date of publication of application: **29.06.2022**
(21) Application number: 20869703.7
(22) Date of filing: 22.09.2020
(51) Int. Cl.: C07K 14/705, A61K 38/17, A61P 31/14, A61P 31/22

(54) **NANO-PERFORATOR HAVING IMPROVED ANTI-VIRAL ACTIVITY**

(30) Priority: 24.09.2019 KR 20190117177
(71) Applicant: Mvrix Co., Ltd., Gyeonggi-do 18469 (KR)
(72) Inventor: KWEON, Dae-Hyuk, Gyeonggi-do 16419 (KR); JUNG, Young-Hun, Gyeonggi-do 16419 (KR); MOON, Seok-Oh, Gyeonggi-do 16419 (KR); OH, Hyun-Suk, Gyeonggi-do 16419 (KR); YU, Seok-Hyun, Gyeonggi-do 16419 (KR); HWANG, Jae-Hyeon, Daegu 42273 (KR)
(74) Representative: Strych, Sebastian
(86) International application number: PCT/KR2020/012738
(87) International publication number: WO 2021/060791

(57) **Abstract**

The present invention relates to improvement in the prevention and treatment of viral infectious diseases through the structural modification or improvement of a nano-perforator. According to the present invention, a nano-perforator, having modified structure, area, shape and membrane scaffold protein characteristics, has improved thermal stability and has maximized anti-viral activity through an increase in the efficiency of perforation activity, an increase in the stability of nano-perforator and the provision of virus specificity, and thus can be usable as a pharmaceutical composition for preventing or treating viral infectious diseases.

<110>    Research and Business Foundation SUNGKYUNKWAN UNIVERSITY

<120>    NANO-PERFORATORNano-perforator HAVING ENHANCED ANTIVIRAL ACTIVITY

<130>    R-2019-0230-KR-1

<160>    6

<170>    KoPatentIn 3.0

<210>    1
<211>    1251
<212>    DNA
<213>    Artificial Sequence

<220>
<223>    CfaC-linker-MSP1E3D1-HisX7-CfaN (esMSP)

<400>    1
gtcaagatca ttagtcgtaa gagtctgggc actcaaaacg tctacgatat tggagtagaa      60

aaagatcata attttttgct gaagaatggg ctggtggcct ctaactgctt caacggtgga      120

ggaggctctg gtggaggcgg tagcggaggc ggagggtcgt ctaccttcag taaacttcgc      180

gaacaactgg gccccgtgac gcaggaattc tgggacaacc tggaaaaaga aaccgaggga      240

ctgcgtcagg aaatgtccaa agatttagaa gaggtgaagg ccaaggttca gccatatctc      300

gatgactttc agaaaaaatg gcaggaagag atggaattat atcgtcaaaa ggtggaaccg      360

ctgcgtgcgg aactgcaaga gggggcacgc caaaaactcc atgagctcca agagaagctc      420

agcccattag gcgaagaaat gcgcgatcgc gcccgtgcac atgttgatgc actccggact      480

catttggcgc catatctcga tgactttcag aaaaaatggc aggaagagat ggaattatat      540

cgtcaaaagg tggaaccgct gcgtgcggaa ctgcaagagg gggcacgcca aaaactccat      600

gagctccaag agaagctcag cccattaggc gaagaaatgc gcgatcgcgc ccgtgcacat      660

gttgatgcac tccggactca tttggcgccg tattcggatg aacttcgcca gcgtttggcc      720

gcacgtctcg aggcgctgaa agaaaacggg ggtgcccgct tggctgagta ccacgcgaaa      780

gcgacagaac acctgagcac cttgagcgaa aaagcgaaac cggcgctgga agatctacgc      840

cagggcttat tgcctgttct tgagagcttt aaagtcagtt ttctgtcagc tctggaagaa      900

tatactaaaa agctgaatac ccagcatcat catcatcatc atcactgcct gtcttacgac      960

acagagattc tgaccgttga atatggattc cttcctatcg gtaagatcgt ggaggaacgg      1020

attgaatgca cagtctatac ggtagataaa aatggctttg tgtatacaca acctattgct      1080

cagtggcata accggggaga acaggaagtt ttcgaatact gcttagaaga cggttcgatt      1140

atccgtgcaa cgaaagatca caaatttatg acgaccgacg gtcagatgtt accgattgat      1200

gagattttcg aacgggggtt agacctgaaa caagttgatg gtttgccgta a              1251

<210>    2
<211>    2016
<212>    DNA
<213>    Artificial Sequence

<220>
<223>    CfaC-linker-MSP1-MSP2-HisX7-CfaN (eslMSP)

<400>    2
atggtcaaga tcattagtcg taagagtctg ggcactcaaa acgtctacga tattggagta      60

gaaaaagatc ataatttttt gctgaagaat gggctggtgg cctctaactg cttcaacggt     120

ggaggaggct ctggtggagg cggtagcgga ggcggagggt cgtctacctt cagtaaactt     180

cgcgaacaac tgggccccgt gacgcaggaa ttctgggaca acctggaaaa agaaaccgag     240

ggactgcgtc aggaaatgtc caaagattta gaagaggtga aggccaaggt tcagccatat     300

ctcgatgact ttcagaaaaa atggcaggaa gagatggaat tatatcgtca aaaggtggaa     360

ccgctgcgtg cggaactgca agagggggca cgccaaaaac tccatgagct ccaagagaag     420

ctcagcccat taggcgaaga aatgcgcgat cgcgcccgtg cacatgttga tgcactccgg     480

actcatttgg cgccatatct cgatgacttt cagaaaaaat ggcaggaaga gatggaatta     540

tatcgtcaaa aggtggaacc gctgcgtgcg gaactgcaag agggggcacg ccaaaaactc     600

catgagctcc aagagaagct cagcccatta ggcgaagaaa tgcgcgatcg cgcccgtgca     660

catgttgatg cactccggac tcatttggcg ccgtattcgg atgaacttcg ccagcgtttg     720

gccgcacgtc tcgaggcgct gaaagaaaac gggggtgccc gcttggctga gtaccacgcg     780

aaagcgacag aacacctgag caccttgagc gaaaaagcga aaccggcgct ggaagatcta     840

cgccagggct tattgcctgt tcttgagagc tttaaagtca gttttctgtc agctctggaa     900

gaatatacta aaaagctgaa tacccagacc ttcagtaaac ttcgcgaaca actgggcccc     960

gtgacgcagg aattctggga caacctggaa aaagaaaccg agggactgcg tcaggaaatg    1020

tccaaagatt tagaagaggt gaaggccaag gttcagccat atctcgatga ctttcagaaa    1080

aaatggcagg aagagatgga attatatcgt caaaaggtgg aaccgctgcg tgcggaactg    1140

caagaggggg cacgccaaaa actccatgag ctccaagaga agctcagccc attaggcgaa    1200

gaaatgcgcg atcgcgcccg tgcacatgtt gatgcactcc ggactcattt ggcgccatat    1260

ctcgatgact ttcagaaaaa atggcaggaa gagatggaat tatatcgtca aaaggtggaa    1320

ccgctgcgtg cggaactgca agagggggca cgccaaaaac tccatgagct ccaagagaag    1380

ctcagcccat taggcgaaga aatgcgcgat cgcgcccgtg cacatgttga tgcactccgg    1440

actcatttgg cgccgtattc ggatgaactt cgccagcgtt tggccgcacg tctcgaggcg    1500

ctgaaagaaa acgggggtgc ccgcttggct gagtaccacg cgaaagcgac agaacacctg    1560

agcaccttga gcgaaaaagc gaaaccggcg ctggaagatc tacgccaggg cttattgcct    1620

gttcttgaga gctttaaagt cagttttctg tcagctctgg aagaatatac taaaaagctg 1680

aatacccagc atcatcatca tcatcatcac tgcctgtctt acgacacaga gattctgacc 1740

gttgaatatg gattccttcc tatcggtaag atcgtggagg aacggattga atgcacagtc 1800

tatacggtag ataaaaatgg ctttgtgtat acacaaccta ttgctcagtg gcataaccgg 1860

ggagaacagg aagttttcga atactgctta gaagacggtt cgattatccg tgcaacgaaa 1920

gatcacaaat ttatgacgac cgacggtcag atgttaccga ttgatgagat tttcgaacgg 1980

gggttagacc tgaaacaagt tgatggtttg ccgtaa 2016

<210> 3
<211> 1185
<212> DNA
<213> Artificial Sequence

<220>
<223> MSP-linker-CfaN

<400> 3
atgggtcatc atcatcatca tcatcacgat tatgatattc ctactactga gaatttgtat 60

tttcagggtt ctaccttcag taaacttcgc gaacaactgg gccccgtgac gcaggaattc 120

tgggacaacc tggaaaaaga aaccgaggga ctgcgtcagg aaatgtccaa agatttagaa 180

gaggtgaagg ccaaggttca gccatatctc gatgactttc agaaaaaatg gcaggaagag 240

atggaattat atcgtcaaaa ggtggaaccg ctgcgtgcgg aactgcaaga gggggcacgc 300

caaaaactcc atgagctcca agagaagctc agcccattag gcgaagaaat gcgcgatcgc 360

gcccgtgcac atgttgatgc actccggact catttggcgc catatctcga tgactttcag 420

aaaaaatggc aggaagagat ggaattatat cgtcaaaagg tggaaccgct gcgtgcggaa 480

ctgcaagagg gggcacgcca aaaactccat gagctccaag agaagctcag cccattaggc 540

gaagaaatgc gcgatcgcgc ccgtgcacat gttgatgcac tccggactca tttggcgccg 600

tattcggatg aacttcgcca gcgtttggcc gcacgtctcg aggcgctgaa agaaaacggg 660

ggtgcccgct tggctgagta ccacgcgaaa gcgacagaac acctgagcac cttgagcgaa 720

aaagcgaaac cggcgctgga agatctacgc cagggcttat tgcctgttct tgagagcttt 780

aaagtcagtt ttctgtcagc tctggaagaa tatactaaaa agctgaatac ccagggtgga 840

ggaggctctg gtggaggcgg tagcggaggc ggagggtcgt gcctgtctta cgacacagag 900

attctgaccg ttgaatatgg attccttcct atcggtaaga tcgtggagga acggattgaa 960

tgcacagtct atacggtaga taaaaatggc tttgtgtata cacaacctat tgctcagtgg 1020

cataaccggg gagaacagga agttttcgaa tactgcttag aagacggttc gattatccgt 1080

gcaacgaaag atcacaaatt tatgacgacc gacggtcaga tgttaccgat tgatgagatt 1140

ttcgaacggg ggttagacct gaaacaagtt gatggtttgc cgtaa 1185

<210> 4
<211> 996
<212> DNA
<213> Artificial Sequence

<220>
<223> CfaC-linker-MSP

<400> 4
atggtcaaga tcattagtcg taagagtctg ggcactcaaa acgtctacga tattggagta 60

gaaaaagatc ataatttttt gctgaagaat gggctggtgg cctctaactg cttcaacggt 120

ggaggaggct ctggtggagg cggtagcgga ggcggagggt cgggtcatca tcatcatcat 180

catcacgatt atgatattcc tactactgag aatttgtatt ttcagggttc taccttcagt 240

aaacttcgcg aacaactggg ccccgtgacg caggaattct gggacaacct ggaaaaagaa 300

accgagggac tgcgtcagga aatgtccaaa gatttagaag aggtgaaggc caaggttcag 360

ccatatctcg atgactttca gaaaaaatgg caggaagaga tggaattata tcgtcaaaag 420

gtggaaccgc tgcgtgcgga actgcaagag ggggcacgcc aaaaactcca tgagctccaa 480

gagaagctca gcccattagg cgaagaaatg cgcgatcgcg cccgtgcaca tgttgatgca 540

ctccggactc atttggcgcc atatctcgat gactttcaga aaaaatggca ggaagagatg 600

gaattatatc gtcaaaaggt ggaaccgctg cgtgcggaac tgcaagaggg ggcacgccaa 660

aaactccatg agctccaaga gaagctcagc ccattaggcg aagaaatgcg cgatcgcgcc 720

cgtgcacatg ttgatgcact ccggactcat ttggcgccgt attcggatga acttcgccag 780

cgtttggccg cacgtctcga ggcgctgaaa gaaaacgggg gtgcccgctt ggctgagtac 840

cacgcgaaag cgacagaaca cctgagcacc ttgagcgaaa aagcgaaacc ggcgctggaa 900

gatctacgcc agggcttatt gcctgttctt gagagcttta aagtcagttt tctgtcagct 960

ctggaagaat atactaaaaa gctgaatacc cagtaa 996

<210> 5
<211> 880
<212> DNA
<213> Artificial Sequence

<220>
<223> FcMSP

<400> 5
nggcggtggc ggtagcggcg gtggcggtag cggcggtggc ggtagcggtc atcatcatca 60

tcatcatcac gattatgata ttcctactac tgagaatttg tattttcagg gttctacctt 120

cagtaaactt cgcgaacaac tgggccccgt gacgcaggaa ttctgggaca acctggaaaa 180

agaaaccgag ggactgcgtc aggaaatgtc caaagattta gaagaggtga aggccaaggt 240

tcagccatat ctcgatgact ttcagaaaaa atggcaggaa gagatggaat tatatcgtca 300

aaaggtggaa ccgctgcgtg cggaactgca agagggggca cgccaaaaac tccatgagct 360

ccaagagaag ctcagcccat taggcgaaga aatgcgcgat cgcgcccgtg cacatgttga 420

tgcactccgg actcatttgg cgccatatct cgatgacttt cagaaaaaat ggcaggaaga 480

gatggaatta tatcgtcaaa aggtggaacc gctgcgtgcg gaactgcaag agggggcacg 540

ccaaaaactc catgagctcc aagagaagct cagcccatta ggcgaagaaa tgcgcgatcg 600

cgcccgtgca catgttgatg cactccggac tcatttggcg ccgtattcgg atgaacttcg 660

ccagcgtttg gccgcacgtc tcgaggcgct gaaagaaaac gggggtgccc gcttggctga 720

gtaccacgcg aaagcgacag aacacctgag caccttgagc gaaaaagcga aaccggcgct 780

ggaagatcta cgccagggct tattgcctgt tcttgagagc tttaaagtca gttttctgtc 840

agctctggaa gaatatacta aaaagctgaa tacccagtaa 880

<210> 6
<211> 1999
<212> DNA
<213> Artificial Sequence

<220>
<223> Fc-eslMSP

<400> 6
   gtcaagatca ttagtcgtaa gagtctgggc actcaaaacg tctacgatat tggagtagaa 60
   aaagatcata attttttgct gaagaatggg ctggtggcct ctaactgctt caacggtgga 120
   ggaggctctn ggaggcggag ggtcgtctac cttcagtaaa cttcgcgaac aactgggccc 180
   cgtgacgcag gaattctggg acaacctgga aaaagaaacc gagggactgc gtcaggaaat 240
   gtccaaagat ttagaagagg tgaaggccaa ggttcagcca tatctcgatg actttcagaa 300
   aaaatggcag gaagagatgg aattatatcg tcaaaaggtg gaaccgctgc gtgcggaact 360
   gcaagagggg gcacgccaaa aactccatga gctccaagag aagctcagcc cattaggcga 420
   agaaatgcgc gatcgcgccc gtgcacatgt tgatgcactc cggactcatt tggcgccata 480
   tctcgatgac tttcagaaaa aatggcagga agagatggaa ttatatcgtc aaaaggtgga 540
   accgctgcgt gcggaactgc aagagggggc acgccaaaaa ctccatgagc tccaagagaa 600
   gctcagccca ttaggcgaag aaatgcgcga tcgcgcccgt gcacatgttg atgcactccg 660
   gactcatttg gcgccgtatt cggatgaact tcgccagcgt ttggccgcac gtctcgaggc 720
   gctgaaagaa aacgggggtg cccgcttggc tgagtaccac gcgaaagcga cagaacacct 780
   gagcaccttg agcgaaaaag cgaaaccggc gctggaagat ctacgccagg gcttattgcc 840
   tgttcttgag agctttaaag tcagttttct gtcagctctg gaagaatata ctaaaaagct 900
   gaatacccag accttcagta aacttcgcga acaactgggc cccgtgacgc aggaattctg 960
   ggacaacctg gaaaaagaaa ccgaggactt gcgtcaggaa atgtccaaag atttagaaga 1020
   ggtgaaggcc aaggttcagc catatctcga tgactttcag aaaaaatggc aggaagagat 1080
   ggaattatat cgtcaaaagg tggaaccgct gcgtgcggaa ctgcaagagg gggcacgcca 1140
   aaaactccat gagctccaag agaagctcag cccattaggc gaagaaatgc gcgatcgcgc 1200
   ccgtgcacat gttgatgcac tccggactca tttggcgcca tatctcgatg actttcagaa 1260
   aaaatggcag gaagagatgg aattatatcg tcaaaaggtg gaaccgctgc gtgcggaact 1320
   gcaagagggg gcacgccaaa aactccatga gctccaagag aagctcagcc cattaggcga 1380
   agaaatgcgc gatcgcgccc gtgcacatgt tgatgcactc cggactcatt tggcgccgta 1440
   ttcggatgaa cttcgccagc gtttggccgc acgtctcgag gcgctgaaag aaaacggggg 1500
   tgcccgcttg gctgagtacc acgcgaaagc gacagaacac ctgagcacct tgagcgaaaa 1560
   agcgaaaccg gcgctggaag atctacgcca gggcttattg cctgttcttg agagctttaa 1620
   agtcagtttt ctgtcagctc tggaagaata tactaaaaag ctgaataccc agcatcatca 1680
   tcatcatcat cactgcctgt cttacgacac agagattctg accgttgaat atggattcct 1740
   tcctatcggt aagatcgtgg aggaacggat tgaatgcaca gtctatacgg tagataaaaa 1800
   tggctttgtg tatacacaac ctattgctca gtggcataac cggggagaac aggaagtttt 1860
   cgaatactgc ttagaagacg gttcgattat ccgtgcaacg aaagatcaca aatttatgac 1920
   gaccgacggt cagatgttac cgattgatga gattttcgaa cgggggttag acctgaaaca 1980
   agttgatggt ttgccgtaa 1999

## Description

### [Technical Field]

The present invention relates to prevention of viral infection and improvement of therapeutic efficacy therefor through structural modification or improvement of nanoperforators.

### [Background Art]

Therapeutic agents for viral infections developed and known to date include amantadine- or rimantadine-type M2 ion channel inhibitors and oseltamivir- (trade name: Tamiflu) or zanamivir (trade name: Relenza)-type neuraminidase inhibitors, but these therapeutic agents have a problem of limited effectiveness. That is, it is known that strain viruses resistant to amantadine- or rimantadine-derived compounds are rapidly produced, H5N1-type influenza viruses detected in some areas are resistant to amantadine- or rimantadine-based compounds, and influenza B virus is insensitive to amantadine derivatives. In addition, it is known that the number of viruses resistant to oseltamivir- or zanamivir-derived compounds also increases, and these resistant viruses frequently occur in children.

Research is being actively conducted to develop a novel therapeutic agent that overcomes the problems with conventional treatment of viral infections as described above. For example, Korean Patent No. 1334143 discloses a composition for preventing or treating colds, avian influenza, swine influenza or swine flu containing a *Polygala karensium* extract and a xanthone-based compound isolated therefrom. However, these agents have low antiviral activity and thus are ineffective in preventing or treating swine flu.

Recently, as a novel application of nanodiscs, the potential thereof as an antiviral agent through, for example, prevention of infection of a virus having a lipid bilayer envelope or inhibition of proliferation of an infected virus has been reported, and the nanodisc is also called a "nanoperforator (NP)". The virus infects host cells using a membrane-bound protein in the lipid bilayer envelope, and the nanoperforator disrupts the infection pathway of the virus or directly punctures the surface of the virus to inhibit the proliferation of the virus. Conventional developed therapeutic agents for viruses, such as amantadine, oseltamivir (trade name Tamiflu), and zanamivir (trade name Relenza), exhibit only limited effects due to tolerance or resistance to the virus. The nanoperforator has advantages of being safe because it does not contain a substance that induces a specific reaction *in vivo*, and being commonly applicable to various strains of viruses, thus actively being researched.

Accordingly, the present inventors have completed the present invention by variously modifying the nanoperforator to further improve antiviral efficacy and increase thermal stability.

### [Disclosure]

### [Technical Problem]

It is one object of the present invention to provide a nanoperforator having improved antiviral activity.

It is another object of the present invention to provide a pharmaceutical composition for preventing or treating viral infections.

It is another object of the present invention to provide a method of producing a nanoperforator.

### [Technical Solution]

In accordance with one aspect of the present invention, the above and other objects can be accomplished by the provision of a nanoperforator containing an end-spliced membrane scaffold protein.

In accordance with another aspect of the present invention, provided is a double nanoperforator including two nanoperforators linked through a covalent bond.

In accordance with another aspect of the present invention, provided is a pharmaceutical composition for preventing or treating viral infections including a nanoperforator having improved antiviral activity.

In accordance with another aspect of the present invention, provided is a method of producing a nanoperforator.

### [Advantageous effects]

The nanoperforator that is modified with regard to structure, area, shape, and membrane scaffold protein according to the present invention has improved thermal stability, and maximized antiviral activity through increased perforation efficiency, increased stability of nanoperforators, and imparted virus specificity, thus being useful for a pharmaceutical composition for preventing or treating viral infections.

### [Description of Drawings]

FIG. 1 is a schematic diagram illustrating the structure of an end-spliced membrane scaffold protein (esMSP) plasmid.
FIG. 2 is a diagram identifying a purified esMSP protein and the end-spliced (cyclic) structure thereof.
FIG. 3 shows a diagram comparing a process of purifying the end-spliced membrane scaffold protein (esMSP) plasmid using sortase with a general esMSP protein purification process, and a graph showing the endotoxin concentration per 1 mg of the esMSP protein produced by the corresponding method, measured through LAL assay (control group ApoA-1 or MSP1E3D1).
FIG. 4 is a diagram illustrating the thermal stability of esMSP.
FIG. 5 is a schematic diagram illustrating the structure of the eslMSP protein.
FIG. 6 is a diagram illustrating a purified eslMSP protein and the end-spliced (cyclic) structure thereof.
FIG. 7 is a diagram identifying the result of trans-splicing of MSP-CfaN and CfaC-MSP,
   wherein the arrow represents a MSP-MSP conjugate.
FIG. 8 is a schematic diagram illustrating the structure of FcMSP capable of binding to an antibody.
FIG. 9 is a diagram identifying a purified MSP (left: 32.6 KDa) and FcMSP including an Fc-binding domain (right: 35.2 KDa).
FIG. 10 is a schematic diagram illustrating the structure of a Fc-eslMSP.
FIG. 11 is a diagram illustrating the results of SDS-PAGE to identify purification of an Fc-eslMSP and formation of an Fc-eslND.
FIG. 12 is a graph illustrating the results of SEC of NP(ND) and esNP(esND).
FIG. 13 illustrates the results of SEC and DLS, and comparison between ND and eslND.
FIG. 14 is a diagram illustrating the results of SEC, SDS-PAGE, and DLS to identify the formation of double nanoperforators (Di-ND or Di-NP).
FIG. 15 is a diagram illustrating the results of SEC and SDS-PAGE of FcND.
FIG. 16 is a diagram illustrating the result of SEC of Fc-eslND.
FIG. 17 is a diagram illustrating the results of DLS and SEC to identify the formation conditions of esdND, which is an end-spliced double nanoperforator including two nanoperforators linked through a disulfide bond, wherein:
   esND-TCEP_Di-form: a disulfide-linked double nanoperforator without addition of a reducing agent during purification of esMSP and formation of ND; and
   after TCEP treat: the result of DLS analysis of esdND, which is a disulfide-linked end-spliced double nanoperforator, after treatment with a reducing agent, TCEP;
   esND-TCEP single-form: a single nanoperforator by addition of a reducing agent during purification of esMSP and formation of ND; and
   after TCEP treat: the result of DLS analysis of esND, which is a single nanoperforator, after treatment with a reducing agent, TCEP.

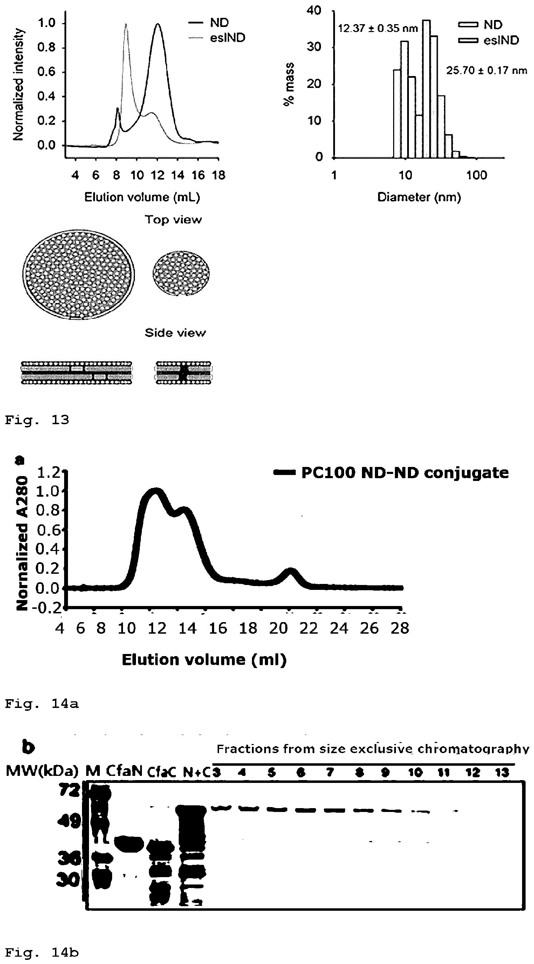

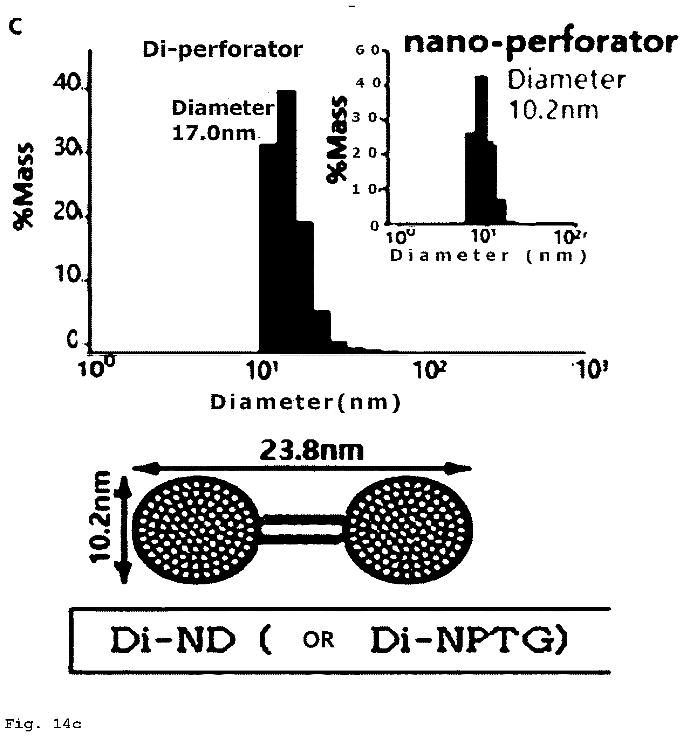

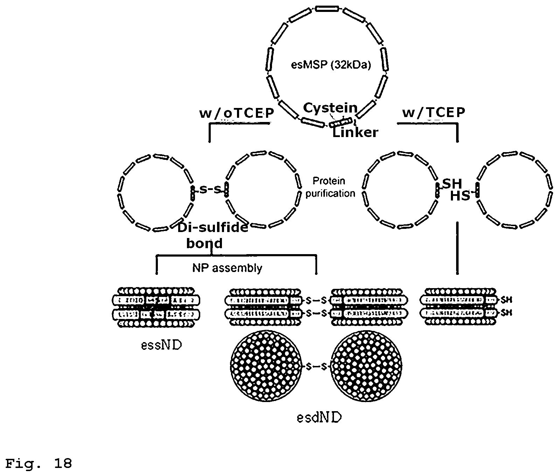

FIG. 18 is a schematic diagram illustrating the production of esND (also called "essND") and esdND summarizing the results of FIG. 14 to identify the formation of esdND, which is a disulfide-bonded double nanoperforator.

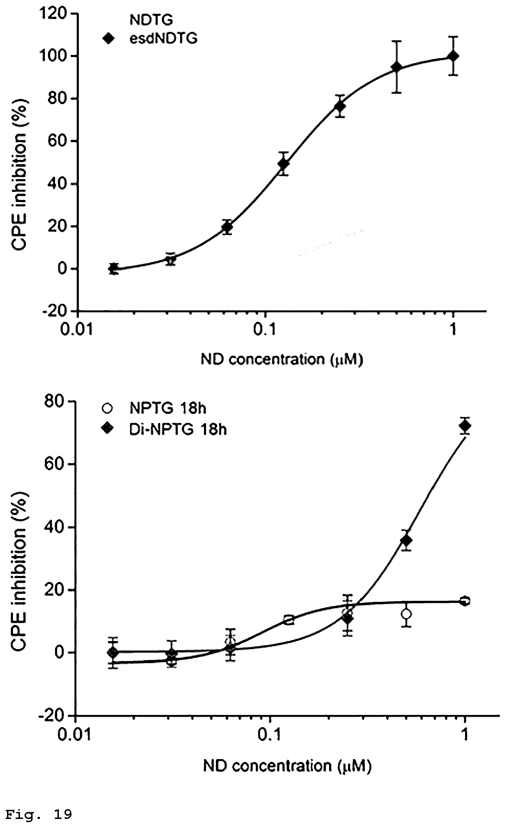

FIG. 19 is a diagram identifying the inhibitory activity of a cytopathic effect (CPE) to identify the improved antiviral efficacy of the end-spliced double nanoperforator, wherein:
   Top: enhanced antiviral efficacy of disulfide-linked end-spliced double nanoperforator (esdNPTG) compared to single nanoperforator, and
   Bottom: enhanced antiviral efficacy of covalently-linked end-spliced double nanoperforator (Di-NPTG) compared to single nanoperforator.

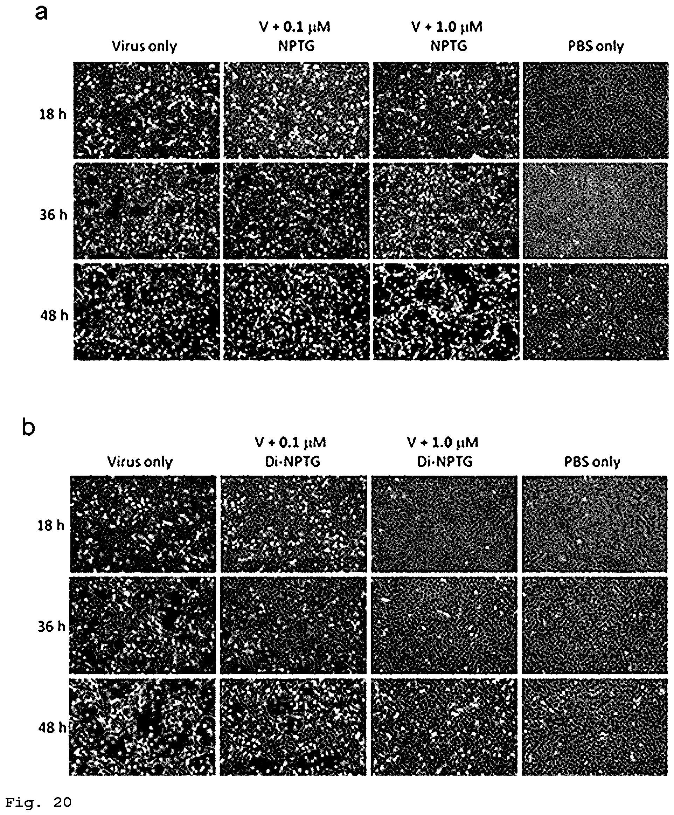

FIG. 20 is a diagram illustrating the effect of the covalently linked end-spliced double nanoperforator (Di-NPTG) on inhibition of viral CPE over time, detected using an optical microscope.

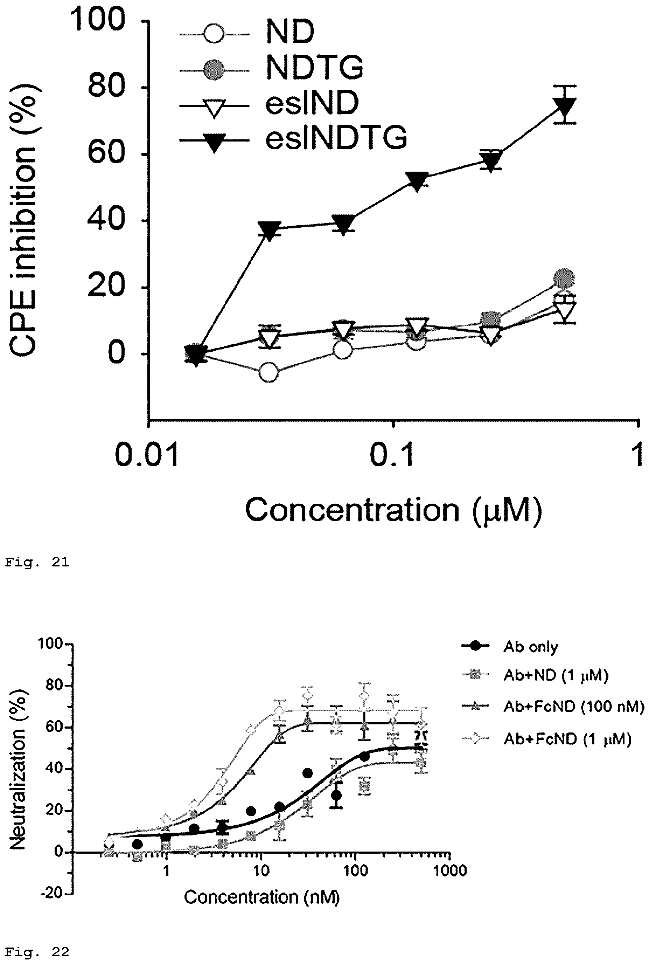

FIG. 21 is a diagram comparing the improved antiviral CPE inhibitory effect of eslNDTG having a larger diameter compared to a small-sized NDTG as a control group.
FIG. 22 is a diagram illustrating the result of a microneutralization assay using FcND.

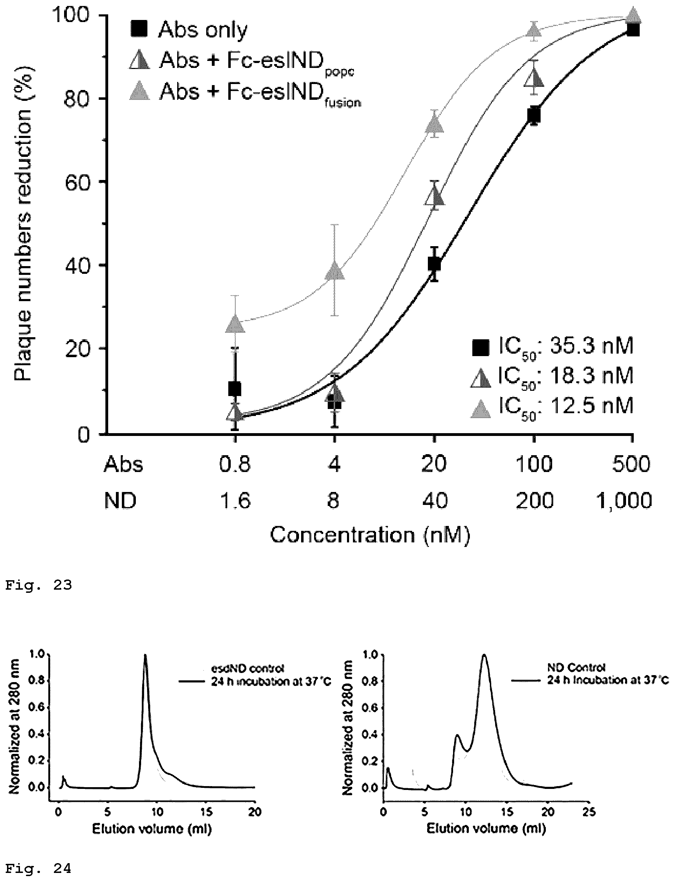

FIG. 23 is a diagram of a plaque reduction assay identifying that PR/8 H1N1 influenza infection is reduced using an antiviral antibody or a mixture of an antiviral antibody with a nanoperforator of each structure.
FIG. 24 is a diagram of size exclusion chromatography (SEC) comparing thermal durability between ND and esdND.

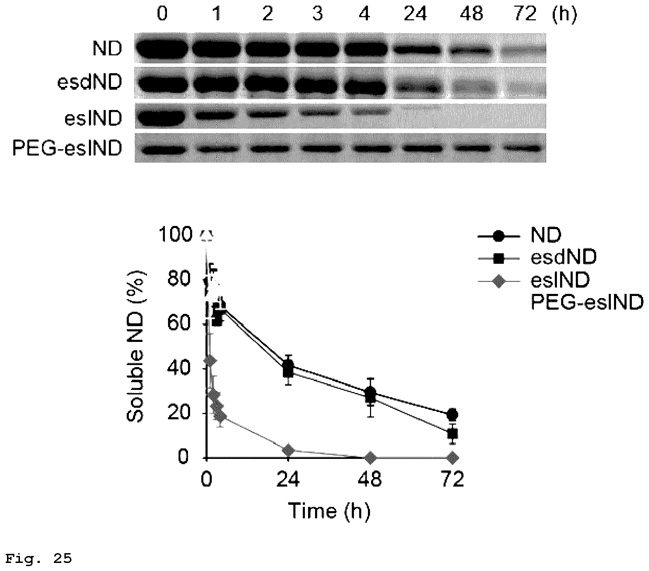

FIG. 25 is a diagram comparing trypsin resistance of ND, esdND, eslND, and PEG-eslND (or PEGylated eslND).

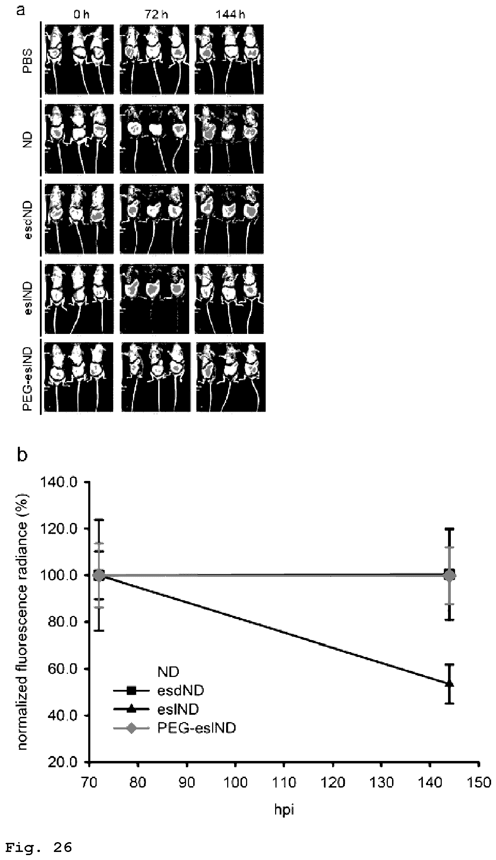

FIG. 26 is a diagram illustrating analysis of the fluorescence values of the distributed nanoperforators remaining in a mouse body over time after injection of the nanoperforators produced from a mixture of a fluorescent dye, wherein:
   a: a diagram of fluorescence value of the nanoperforator of the entire mouse body; and
   b: the result of comparison of fluorescence values between the nanoperforators of respective structures over time based on the calculated chest fluorescence values of mice over time after injection.

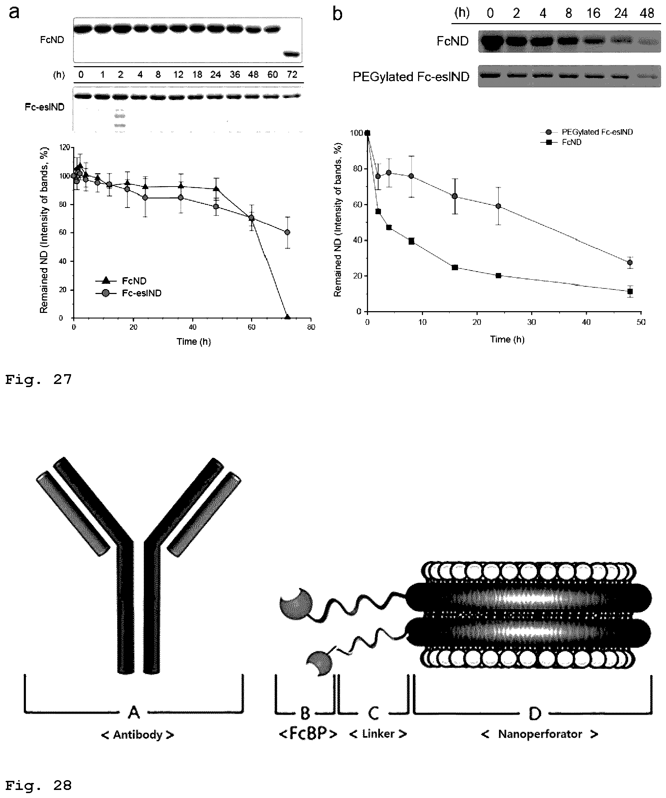

FIG. 27 is a diagram comparing the stability between FcND, Fc-eslND and PEGylated Fc-eslND, wherein:
   a: a comparison of thermal durability between FcND and Fc-eslND; and
   b: a comparison of trypsin resistance between FcND and PEGlyated Fc-eslND.
FIG. 28 is a schematic diagram illustrating a method for measuring binding of an antibody to an antibody-binding nanoperforator.

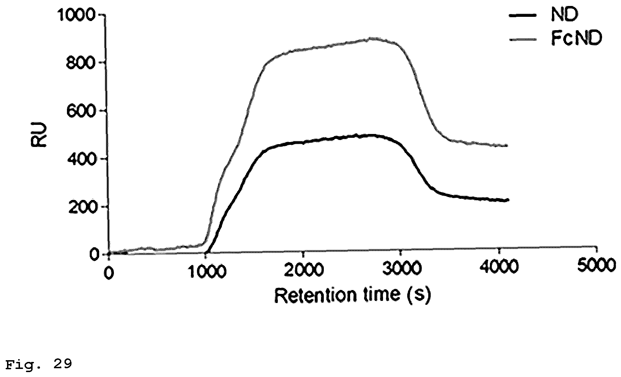

FIG. 29 is a diagram analyzing the binding pattern of the antibody to FcND using surface plasma resonance.

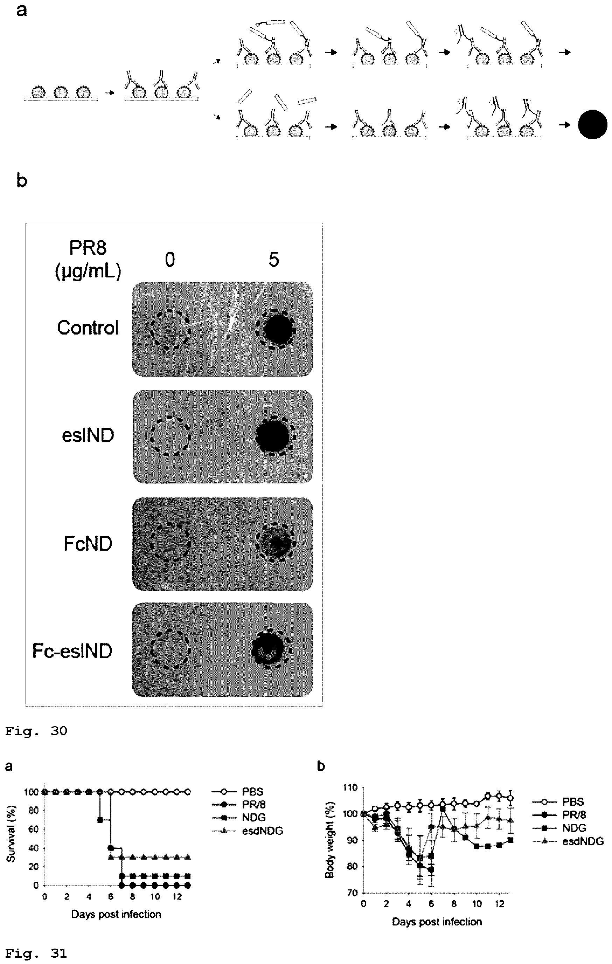

FIG. 30 is a diagram analyzing the antibody-binding pattern of FcND and Fc-eslND using a dot blot assay, wherein:
   a: a schematic diagram of experimental procedure of dot blot assay; and
   b: experimental result of dot blot assay.
FIG. 31 is a diagram illustrating survival rate and body weight changes over time after infection when NPG or esdNPG is injected at a concentration of 0.5 mg/kg into a mouse body after infection with PR/8 H1N1 influenza.

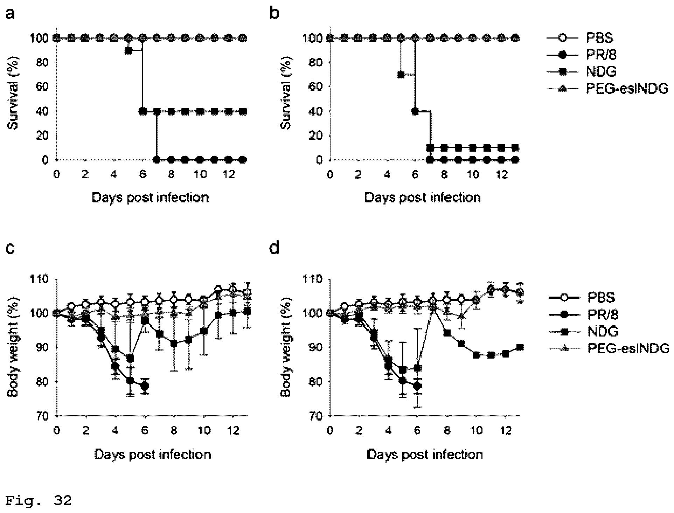

FIG. 32 is a diagram illustrating survival rate and body weight changes over time after infection when NPG or PEG-eslNPG is injected at a concentration of 2.5 mg/kg or 0.5 mg/kg into mice after infection with PR/8 H1N1 influenza.

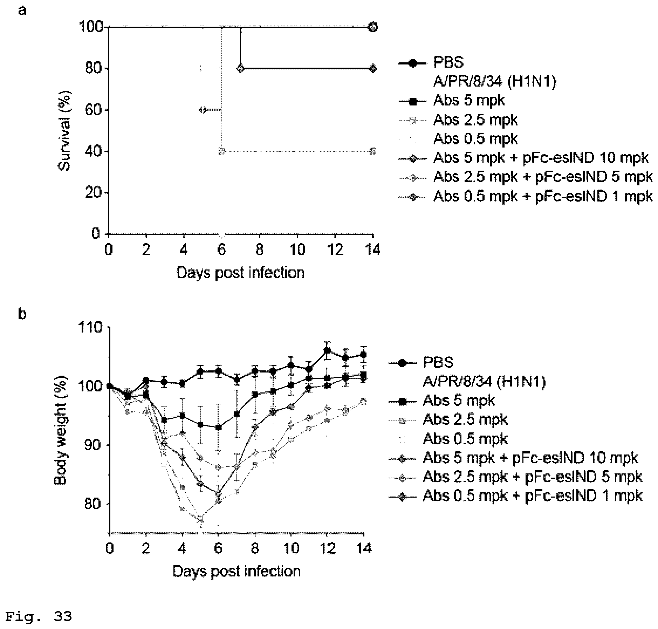

FIG. 33 is a diagram illustrating survival rate and body weight changes over time after infection when an antiviral antibody or a mixture of an antiviral antibody with pFc-eslNP is injected into a mouse body after infection with PR/8 H1N1 influenza.

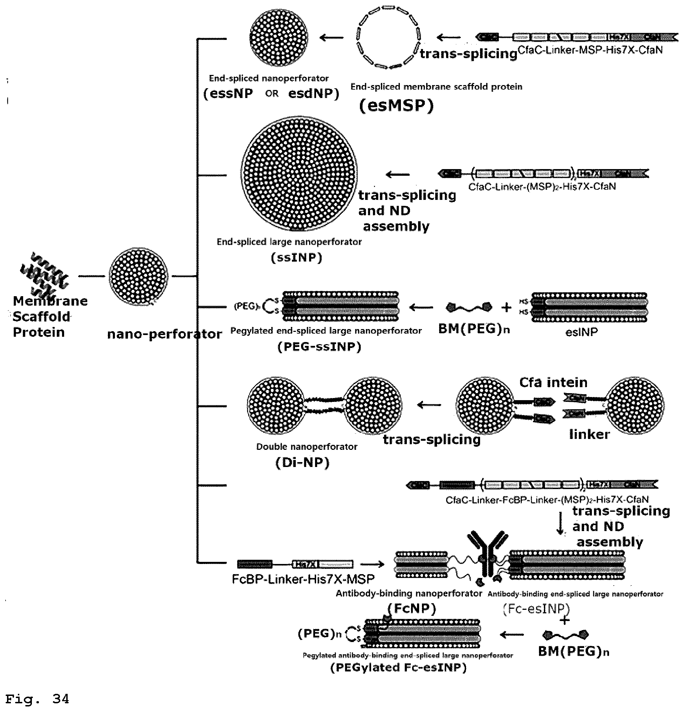

FIG. 34 is a schematic diagram illustrating the sequences and structures of various nanoperforators provided in the present invention.

### [Best Mode]

Hereinafter, embodiments of the present invention will be described in detail with reference to the accompanying drawings. However, the following embodiments are provided merely for illustration of the present invention. Detailed descriptions of technologies and configurations well-known to those skilled in the art will be omitted when they may obscure the subject matter of the present invention, and are not intended to limit the invention. Various modifications and applications of the present invention are possible within the scope of the claims described later and equivalents derived therefrom.

In addition, the terminology used herein is used to accurately describe preferred embodiments, and may be changed according to intentions or customs of users or operators. Accordingly, the definitions of the terminology should be understood based on the content throughout the specification. It will be further understood that terms "comprises" and the like, when used in this specification, specify the presence of other components, but do not preclude the presence or addition of other components unless otherwise mentioned.

Unless otherwise defined, all technical terms used herein have the same meanings as generally understood by those skilled in the art to which the present invention pertains. In addition, preferred methods and samples described herein and equivalents thereto fall within the scope of the present invention. The disclosures of all publications mentioned herein are incorporated herein by reference in their entireties.

The present invention makes reference to Patent Application No. 10-2017-0089655 previously filed by the inventors of the present invention, and the disclosure including the sequence thereof is incorporated herein by reference.

In one aspect, the present invention is directed to a plasmid including a nucleotide sequence encoding a fusion protein in which an N-terminal domain of an intein is fused to a C-terminus of a membrane scaffold protein and a C-terminal domain of the intein is fused to an N-terminus of the membrane scaffold protein.

In an embodiment, the MSP may be MSP1 or MSP2.

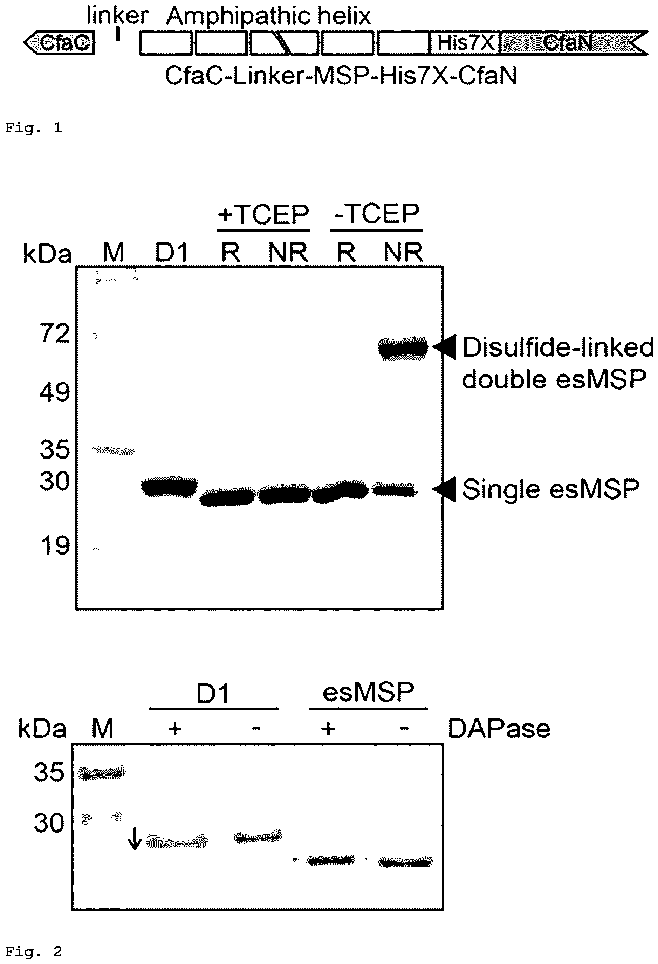

In an embodiment, the fusion protein may sequentially include the C-terminal domain of the intein, a linker, the membrane scaffold protein, and the N-terminal domain of the intein, and the nucleotide sequence encoding the same may have the structure of FIG. 1, and may include the nucleotide sequence of SEQ ID NO: 1.

In an embodiment, the linker may be encoded by a nucleic acid including the sequence of GGGGSGGGGSGGGGS.

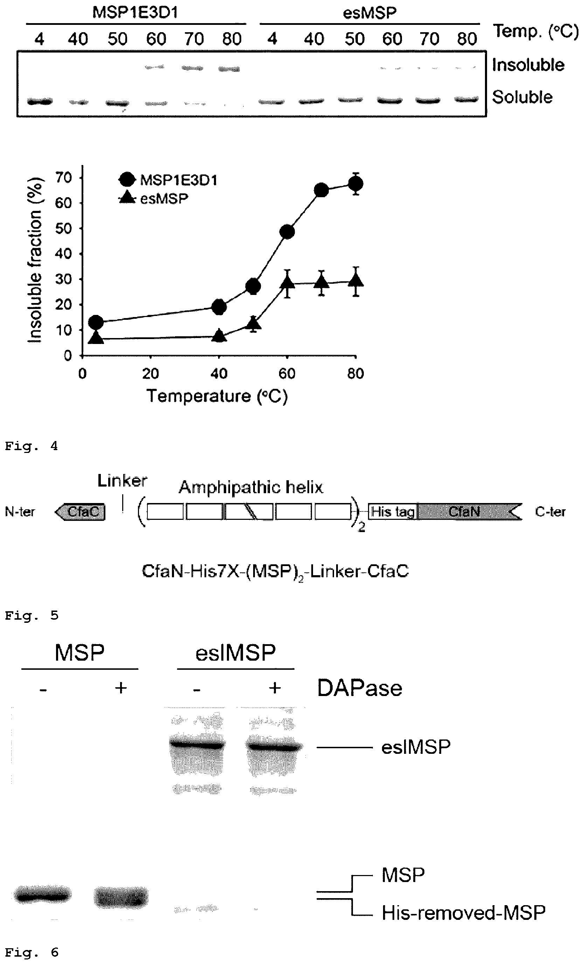

In an embodiment, the fusion protein may include two or more repeated membrane scaffold proteins, and may sequentially include the C-terminal domain of the intein, two or more membrane scaffold proteins, and the N-terminal domain of the intein, and the nucleotide sequence encoding the same may have the structure of FIG. 5 and may include the nucleotide sequence of SEQ ID NO: 2.

In another aspect, the present invention is directed to a plasmid including a nucleotide sequence encoding a fusion protein in which a C-terminal domain of an intein is fused to an N-terminus of a membrane scaffold protein, or a plasmid including a nucleotide sequence encoding a fusion protein in which an N-terminal domain of an intein is fused to a C-terminus of a membrane scaffold protein.

In an embodiment, the plasmid may be a plasmid for producing a double nanoperforator including two nanoperforators linked through a covalent bond.

In another aspect, the present invention is directed to a plasmid including a nucleotide sequence encoding a fusion protein of an Fc region-binding peptide (Fcbp) and a membrane scaffold protein.

In an embodiment, an N-terminal domain of an intein may be fused to a C-terminus of the fusion protein, a C-terminal domain of the intein may be fused to the N-terminus of the fusion protein, and the fusion protein may sequentially include the C-terminal domain of the intein, a linker, the Fc region-binding peptide, a linker, the membrane scaffold protein, and the N-terminal domain of the intein.

In an embodiment, the MSP may be MSP1 or MSP2.

In an embodiment, the linker may be encoded by a nucleic acid including the sequence of GGGGSGGGGSGGGGS.

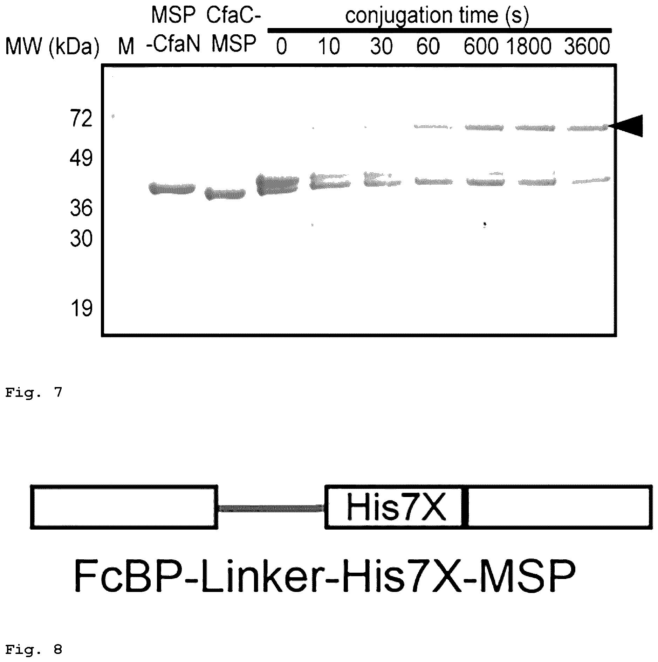

In an embodiment, the plasmid may include the structure of FIG. 8 and may include the nucleotide sequence of SEQ ID NO: 5 in which the FcBP portion is represented by N.

In another aspect, the present invention is directed to a plasmid including a nucleotide sequence encoding a fusion protein of an Fc region-binding peptide and two or more repeated membrane scaffold proteins, wherein an N-terminal domain of an intein is fused to a C-terminus of the fusion protein and a C-terminal domain of the intein is fused to an N-terminus of the fusion protein.

In an embodiment, the fusion protein may sequentially include the C-terminal domain of the intein, the Fc region-binding peptide, two or more membrane scaffold proteins, and the N-terminal domain of the intein.

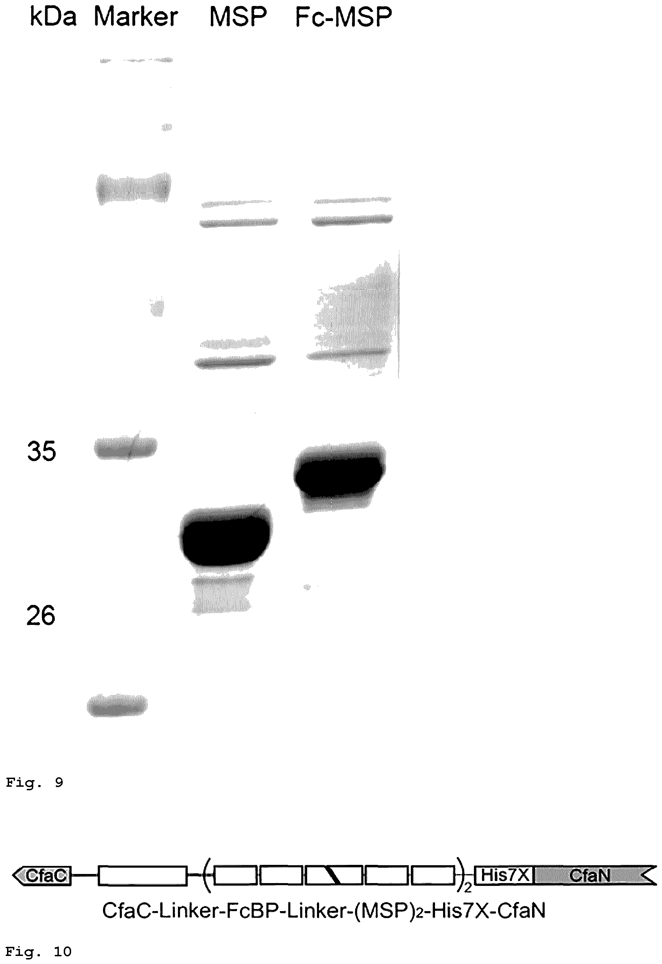

In an embodiment, the plasmid may include the structure of FIG. 10, and may include the nucleotide sequence of SEQ ID NO: 6, wherein the FcBP portion is represented by N.

In another aspect, the present invention is directed to a plasmid including a nucleotide sequence encoding a fusion protein of an Fc region-binding peptide and a membrane scaffold protein, wherein a C-terminal domain of an intein is fused to an N-terminus of the fusion protein.

In another aspect, the present invention is directed to a plasmid including a nucleotide sequence encoding a fusion protein of an Fc region-binding peptide and a membrane scaffold protein, wherein an N-terminal domain of an intein is fused to a C-terminus of the fusion protein.

In an embodiment, the plasmid may be a plasmid for producing a double nanoperforator including two nanoperforators linked through a covalent bond.

In another aspect, the present invention is directed to a membrane scaffold protein for a nanoperforator.

In another aspect, the present invention is directed to an end-spliced membrane scaffold protein (esMSP) having an N-terminus and a C-terminus that are joined together, and encoded by the plasmid of the present invention.

In an embodiment, the end-spliced membrane scaffold protein (esMSP) of the present invention may form a cycle when the intein formed by joining the N-terminal domain of the intein to the C-terminal domain of the intein is removed from the fusion protein of the present invention, whereby the two termini are linked together.

In an embodiment, the end-spliced membrane scaffold protein of the present invention can be obtained by expressing a plasmid encoding a membrane scaffold protein having an intein fused to the terminus thereof and performing protein purification by treatment with a reducing agent.

In an embodiment, it was found that the end-spliced membrane scaffold protein (esMSP) of the present invention has a lower endotoxin concentration and higher thermal stability than a linear membrane scaffold protein (MSP).

In another aspect, the present invention is directed to a double end-spliced membrane scaffold protein, wherein two end-spliced membrane scaffold proteins are linked by a disulfide bond.

In an embodiment, the double end-spliced membrane scaffold protein of the present invention can be obtained by expressing a plasmid encoding an end-spliced membrane scaffold protein having an intein fused to the terminus thereof and performing protein purification without treatment with a reducing agent.

In another aspect, the present invention is directed to an end-spliced large membrane scaffold protein including two or more membrane scaffold proteins that are contiguously linked.

In an embodiment, the end-spliced large membrane scaffold protein may have a greater size than that of the end-spliced membrane scaffold protein of the present invention, and may have a diameter 1.2 to 5 times greater than that of the end-spliced membrane scaffold protein.

In an embodiment, the end-spliced large membrane scaffold protein may be obtained by expressing a plasmid including a nucleotide sequence encoding two or more repeated end-spliced membrane scaffold proteins of the present invention, followed by purification.

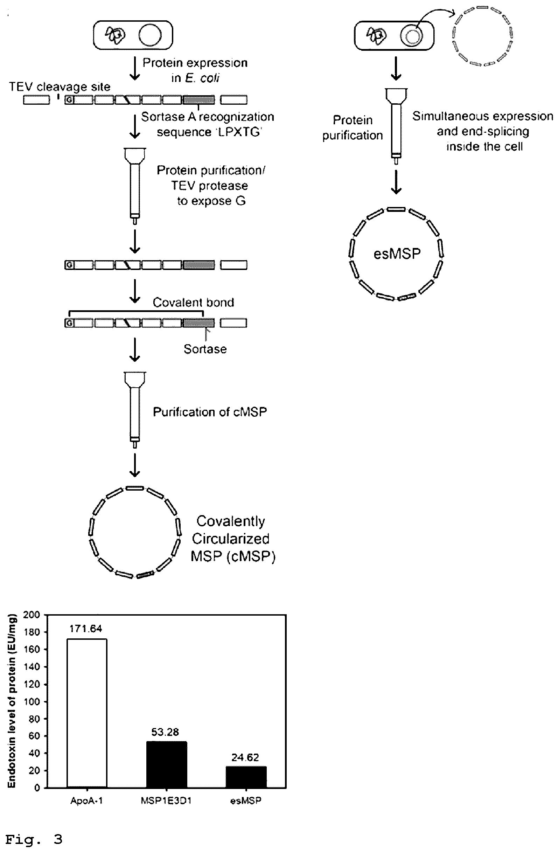

In an embodiment, the end-spliced membrane scaffold proteins of the present invention may form a cycle when both termini are covalently linked (the green part in FIG. 3) using trans-intein splicing for removing a complete intein formed by joining the N-terminal domain of the intein to the C-terminal domain of the intein, and the term "trans-intein splicing" refers to a procedure in which an N-terminal domain of an intein is joined to a C-terminal domain of the intein to form a complete intein complex, the intein complex is removed by splicing, and extein domains (both termini of the fusion domain) outside the intein complex are joined through peptide bonds.

In another aspect, the present invention is directed to a fusion protein in which a C-terminal domain of an intein is fused to an N-terminus of a membrane scaffold protein, obtained by expressing a plasmid including a nucleotide sequence encoding the fusion protein in which the C-terminal domain of the intein is fused to the N-terminus of the membrane scaffold protein, followed by purification, and a fusion protein in which an N-terminal domain of an intein is fused to a C-terminus of a membrane scaffold protein, obtained by expressing a plasmid including a nucleotide sequence encoding the fusion protein in which the N-terminal domain of the intein is fused to the C-terminus of the membrane scaffold protein, followed by purification.

In another aspect, the present invention is directed to a membrane scaffold protein (FcBP-MSP) capable of binding to an antibody (also referred to as "antibody-binding membrane scaffold protein), obtained by expressing the plasmid of the present invention, followed by purification.

In an embodiment, the antibody-binding membrane scaffold protein (FcBP-MSP) may be an end-spliced membrane scaffold protein capable of binding to an antibody, in which the N-terminal domain of the intein is joined to the C-terminus of the fusion protein of the Fc region-binding peptide and the membrane scaffold protein, and the C-terminus of the intein is fused to the N-terminus of the fusion protein to link the N-terminal domain of the fusion protein to the C-terminal domain thereof to thereby form a cycle.

In an embodiment, the antibody-binding end-spliced membrane scaffold protein may be referred to as an "an antibody-binding end-spliced fusion protein" (esFcBP-MSP) .

In an embodiment, the antibody-binding membrane scaffold protein may form a cycle when both termini are joined by removing an intein fragment formed by fusing the N-terminal domain of the intein to the C-terminus of the fusion protein of the Fc region-binding peptide and the membrane scaffold protein, and fusing the C-terminus of the fusion protein to the N-terminus of the fusion protein.

In an embodiment, the antibody-binding end-spliced membrane scaffold protein of the present invention has a lower endotoxin concentration and higher thermal stability than that of a linear membrane scaffold protein (FcBP-MSP).

In an embodiment, the antibody-binding membrane scaffold protein (FcBP-MSP) may be obtained by performing purification by treatment with a reducing agent.

In another aspect, the present invention is directed to an antibody-binding double end-spliced membrane scaffold protein including two antibody-binding end-spliced membrane scaffold proteins that are linked by a disulfide bond.

In an embodiment, the antibody-binding double end-spliced membrane scaffold protein of the present invention can be obtained by expressing the plasmid according to the present invention including a nucleotide sequence encoding the fusion protein in which the N-terminal domain of the intein is fused to the C-terminus of the membrane scaffold protein and the C-terminal domain of the intein is fused to the N-terminus of the membrane scaffold protein, and purifying the protein without treatment with a reducing agent.

In another aspect, the present invention is directed to an antibody-binding end-spliced large membrane scaffold protein, wherein the end-spliced large membrane scaffold protein is encoded by a plasmid including a nucleotide sequence encoding a fusion protein of an Fc region-binding peptide and two or more repeating membrane scaffold proteins, in which the N-terminal domain of the intein is fused to the C-terminus thereof and the C-terminal domain of the intein is fused to the N-terminus thereof, and has an increased size compared to that of the antibody-binding end-spliced membrane scaffold protein.

In an embodiment, the antibody-binding end-spliced large membrane scaffold protein (Fc-eslMSP) may be larger than the antibody-binding end-spliced membrane scaffold protein of the present invention and may have a diameter of 1.2 to 5 times greater than that of the antibody-binding end-spliced membrane scaffold protein (Fc-esMSP).

In an embodiment, the antibody-binding end-spliced large membrane scaffold protein may be obtained by expressing the plasmid including a nucleotide sequence encoding two or more repeated membrane scaffold proteins according to the present invention, followed by purification.

In another aspect, the present invention is directed to a fusion protein of an Fc region-binding peptide and a membrane scaffold protein in which a C-terminal domain of an intein is fused to an N-terminus of the fusion protein, wherein the fusion protein is encoded by a plasmid including a nucleotide sequence encoding the fusion protein of the Fc region-binding peptide and the membrane scaffold protein, wherein the plasmid has a structure in which the C-terminal domain of the intein is fused to the N-terminus of the fusion protein.

In another aspect, the present invention is directed to a fusion protein of an Fc region-binding peptide and a membrane scaffold protein wherein an N-terminal domain of an intein is fused to a C-terminus of the fusion protein, wherein the fusion protein is encoded by a plasmid including a nucleotide sequence encoding the fusion protein of the Fc region-binding peptide and the membrane scaffold protein, wherein the plasmid has a structure in which the N-terminal domain of the intein is fused to the C-terminus of the fusion protein.

In an embodiment, the antibody-binding end-spliced membrane scaffold proteins according to the present invention may form a cycle when both termini are covalently linked (green part in FIG. 3) through trans-intein splicing for removing the complete intein formed by joining the N-terminal domain of the intein of the fusion protein to the C-terminal domain of the intein, and the term "trans-intein splicing" refers to a phenomenon in which the N-terminal domain of the intein is joined to the C-terminal domain of the intein to form a complete intein complex, the intein complex is removed by splicing, and the extein domains (both termini of the fusion domain) outside the intein complex are joined via peptide bonds.

As used herein, the term "intein" refers to a fragment that is cleaved out in the protein-splicing process, which is a post-translational process to remove a part of a protein precursor before the protein is folded into a complete form. It is known that in some proteins, after translation, a part of the precursor protein is cleaved off by autocatalysis and the remaining part is recombined into a mature protein. At this time, the cleaved part is called an "intein", and the part remaining as a mature protein is called an "extein".

In an embodiment, the end-sliced membrane scaffold protein of the present invention may include an intein domain at a terminus thereof, and thus may be assembled into a cyclic form or may be produced into a cyclic form using sortase, without an intein.

In an embodiment, the antibody-binding end-sliced membrane scaffold protein of the present invention may include an intein domain at a terminus thereof, and thus may be assembled into a cyclic form or may be produced into a cyclic form using sortase, without an intein.

In an embodiment, it is possible to improve the protein engineering of a membrane scaffold protein, or a fusion protein in which an Fc-region binding peptide is fused to the membrane scaffold protein, which is to be included in the nanoperforator, using the following method:
1) Protein binding using sortase A: this is an irreversible binding reaction using sortase A, which is a transpeptidase derived from bacteria, where sortase A recognizes a specific amino acid sequence of LPXTG, cleaves a protein substrate between threonine and glycine residues, and binds the result to the cell surface. This is widely used in protein engineering, and imparts an LPXTG sequence and two or more consecutive glycine sequences to two proteins to be bound to facilitate binding between the proteins. Sortases applicable to protein engineering of nanoperforators may be derived from *Sulfobacillus, Rubrobacter, Peptoniphilus, Shewanella, Faecalibaculum, Colwellia, Staphylococcus, Methylorubrum, Gaiella, Bacillus, Clostridium, Blautia, Enterococcus, Streptococcus, Streptomyces, Lactobacillus, Listeria, Pediococcus,* or *Corynebacterium,* but the disclosure is not limited thereto.
2) Trans-intein splicing: a protein domain or peptide fragment necessary for joining the C-terminus of the fusion protein to the N-terminus thereof was added along with a linker. Among them, the protein domain was a trans-intein belonging to the genus DnaE or DnaB set forth in Table 1, such as Cfa, Npu, Ssp, Rma, or Ppu, but is not limited thereto. These trans-inteins are expressed in separate forms, but the N-terminal domain (Int-C) binds to the C-terminal domain (Int-N) to form a complete intein complex and then the intein complex is spliced and detached, and at this time, exteins located outside the intein complex are linked with a peptide bond to form a single protein.

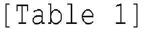

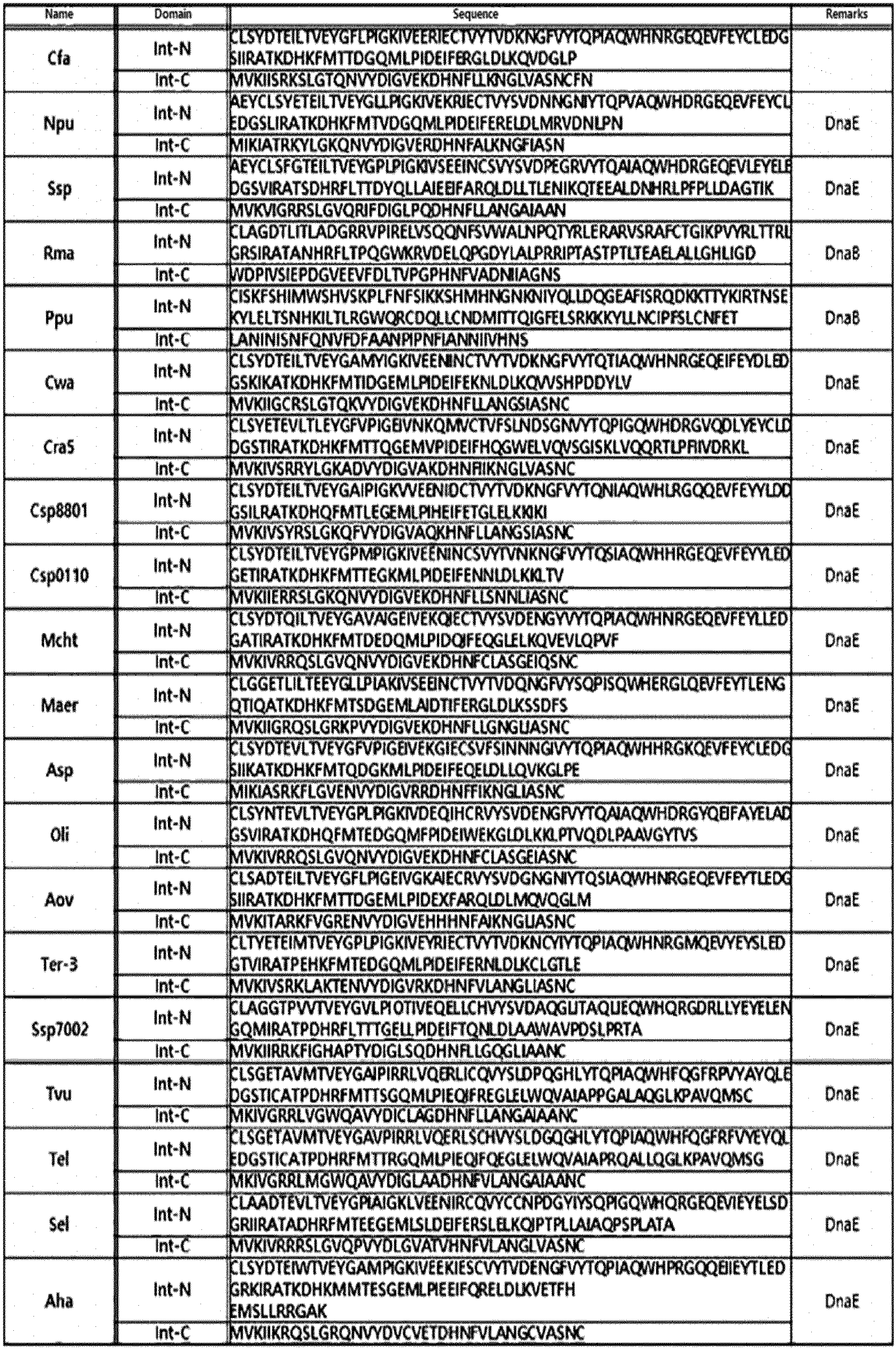

Both of the above methods may be used to produce the end-spliced membrane scaffold protein or the antibody-binding end-spliced membrane scaffold protein according to the present invention. However, the method used in the following examples according to the present invention has the advantage of overcoming problems such as enzyme cost, yield, and speed compared to the method of separately producing end-spliced MSP *in vitro* using sortase because an end-spliced MSP protein is derived from *E. coli* through trans-intein splicing (see FIG. 3). In addition, esMSP produced in recombinant *E. coli* using the method of FIG. 3 of the present invention has endotoxins attached in an amount of 1/7 that of apolipoprotein A1, which is the prototype of MSP, and endotoxins attached in an amount of 1/2.5 that of general MSP, and thus has an advantage of preventing various side effects due to the presence of endotoxins.

In another aspect, the present invention is directed to a nanoperforator including the end-spliced membrane scaffold protein (esMSP) according to the present invention.

In an embodiment, both termini of the membrane scaffold protein may be joined using sortase.

In an embodiment, the nanoperforator of the present invention may be pegylated to improve the stability of the construct, a degrading enzyme resistance, and biostability.

In an embodiment, the nanoperforator of the present invention may include a lipid bilayer nanodisc and a membrane scaffold protein surrounding the outer circumferential surface of the lipid bilayer nanodisc, and may further include a receptor for a surface antigen of a virus.

In an embodiment, the nanoperforator is capable of perforating the lipid bilayer envelope of the virus.

In an embodiment, the virus may be a virus having a lipid bilayer envelope (or membrane), and may include at least one selected from the group consisting of the families *Coronaviridae, Bunyaviridae, Filoviridae, Flaviviridae, Hepadnaviridae, Herpesviridae, Orthomyxoviridae, Poxviridae, Rhabdoviridae, Retroviridae* and *Togaviridae,* and is more preferably an influenza virus.

In an embodiment, it was found that the nanoperforator using the membrane scaffold protein in which both termini of the amino terminus (N-terminus) and the carboxyl terminus (C-terminus) of the membrane scaffold protein constituting the nanoperforator are joined through a covalent bond to form a cyclic structure is better able to resist structural damage due to heat and aggregation at high concentrations than the conventional nanoperforator using the linear membrane scaffold protein. The method for joining both termini of a protein in a cyclic form may also include other protein-engineering techniques, including a method using trans-intein splicing.

In the present invention, the nanoperforator including the end-spliced membrane scaffold protein can improve the antiviral efficacy of the nanoperforator. The two termini of the end-spliced membrane scaffold protein constituting the end-spliced nanoperforator are joined to each other, so the end-spliced nanoperforator is more resistant to heat, and is more stable and resistant to aggregation at a constant size and high concentration than conventional nanoperforators, so degradation or aggregation due to the temperature of the biological subject receiving treatment and due to proteases is reduced, and antiviral efficacy is improved compared to conventional nanoperforators. In addition, it was found that the end-spliced membrane scaffold protein using the trans-intein had a lower endotoxin level than the conventional linear membrane scaffold protein and apolipoprotein, in particular 1/10 of that of the apolipoprotein. This can remarkably reduce the possibility of contamination of the end-spliced membrane scaffold protein using the end-spliced nanoperforator with endotoxins, which cause side effects of high fever, headache, and sepsis (FIG. 3).

In another aspect, the present invention is directed to a nanoperforator including the end-spliced large membrane scaffold protein according to the present invention.

In one embodiment, the nanoperforator of the present invention may be pegylated to improve the stability of the construct and a degrading enzyme resistance.

In an embodiment, when PEG is attached to the nanoperforator of the present invention, the thermal stability of the nanoperforator is remarkably improved, and at the same time, resistance to protease (e.g., trypsin) is greatly increased. Proteases such as trypsin destroy the structure of the nanoperforator and thus loses the antiviral activity thereof. The PEG-attached nanoperforator, having resistance thereto, has excellent antiviral activity.

In an embodiment, the nanoperforator of the present invention may include a lipid bilayer nanodisc and a membrane scaffold protein surrounding the outer circumferential surface of the lipid bilayer nanodisc, and may further include a receptor for a surface antigen of a virus.

In an embodiment, the end-spliced large membrane scaffold protein may be an end-spliced membrane scaffold protein in which two or more membrane scaffold proteins are fused and linked, and the nanoperforator including the same is a nanoperforator (or end-spliced large ND, eslND) having an increased surface area of 15 nm or more in diameter.

In an embodiment, it was found that when two or more membrane scaffold proteins are linked in a cyclic structure to thereby increase the area of the nanoperforator, the ability to damage the virus envelope is improved, the particle sizes of the nanoperforators using the membrane scaffold protein are uniform, and the antiviral effect such as an effect of preventing infection of a virus having a lipid bilayer envelope or an effect of inhibiting the proliferation of the virus, is improved.

In the present invention, it was found that the end-spliced large nanoperforator having an increased size due to linking of two or more membrane scaffold proteins facilitated the perforation effect through fusion with the viral membrane, thereby improving the antiviral effect. A membrane scaffold protein gene was inserted into the plasmid for the end-spliced nanoperforator and was produced using an overlap cloning method, but is not limited thereto. In addition, various membrane scaffold proteins are applicable, without limitation to the MSP1E3D1 protein used in the present invention.

In one embodiment, the end-spliced nanoperforator may have a diameter of 10 to 100 nm, more preferably 15 to 50 nm, but the size of the end-spliced nanoperforator is not limited, so long as the function of the end-spliced nanoperforator using a membrane scaffold protein using a trans-intein can be realized.

In another aspect, the present invention is directed to a double nanoperforator including the double end-spliced membrane scaffold protein according to the present invention.

In an embodiment, the double nanoperforator may have a structure in which two nanoperforators are linked through a disulfide bond.

In an embodiment, the nanoperforator of the present invention may be pegylated to improve the stability of the construct and thus resistance to a degrading enzyme.

In an embodiment, the double nanoperforator of the present invention may include a lipid bilayer nanodisc and a membrane scaffold protein surrounding the outer circumferential surface of the lipid bilayer nanodisc, and may further include a receptor for a surface antigen of a virus, wherein the two nanoperforators may include different receptors for virus surface antigens.

In another aspect, the present invention is directed to a double nanoperforator in which two nanoperforators are linked through a covalent bond, wherein the double nanoperforator has a structure in which a nanoperforator containing the fusion protein according to the present invention, in which the C-terminal domain of the intein is fused to the N-terminus of the membrane scaffold protein, is linked through a covalent bond to a nanoperforator containing the fusion protein according to the present invention, in which the N-terminal domain of the intein is fused to the C-terminus of the membrane scaffold protein.

In an embodiment, the fusion protein, in which the C-terminal domain of the intein is fused to the N-terminus of the membrane scaffold protein, may be linked through a covalent bond to the fusion protein in which the N-terminal domain of the intein is fused to the C-terminus of the membrane scaffold protein.

In an embodiment, the two nanoperforators may be linked through a covalent bond when the intein formed by joining the N-terminal domain of the intein to the C-terminal domain of the intein in the two nanoperforators is removed, wherein the covalent bond is a disulfide bond.

In an embodiment, the membrane scaffold protein of each of the two nanoperforators may include a membrane scaffold protein in which two or more membrane scaffold proteins are repeatedly linked.

In an embodiment, the double nanoperforator may be prepared using a method such as a disulfide bond, a thioether bond, a sortase bond, an intein bond, or the like.

In an embodiment, the double nanoperforator of the present invention may be pegylated to improve the stability of the construct and thus resistance to a degrading enzyme.

In an embodiment, the double nanoperforator of the present invention may include a lipid bilayer nanodisc and a membrane scaffold protein surrounding the outer circumferential surface of the lipid bilayer nanodisc, and may further include a receptor for a surface antigen of a virus, and the two nanoperforators may include different receptors for virus surface antigens.

In an embodiment, trans-intein splicing described later was used to provide the double nanoperforator structure in which two nanoperforators are linked. A receptor having 2,3 sialic acid is inserted into the nanoperforator to which the N-terminal domain of the intein (Int-N) is linked, and a receptor having 2,6 sialic acid is inserted into the nanoperforator to which Int-C is linked, to produce two types of nanoperforators having two different receptors. The combination of the receptor and the nanoperforator having a trans-intein domain is not limiting. A double nanoperforator structure in which the two nanoperforators are linked in a single form through trans-splicing was also produced. The effect of the nanoperforator or double nanoperforator structure on inhibition of influenza virus infection in MDCK cells was determined through CPE inhibition analysis. The result showed that the produced double nanoperforator exhibited antiviral activity against several influenza viruses described above, and the antiviral activity was much better than that of a single nanoperforator. The method of linking the two nanoperforators is also not limited to trans-splicing, and the double nanoperforator may be produced using a method such as a sortase-mediated bond, a disulfide bond, or a maleimide-mediated thioether bond. Also, in the present invention, the membrane scaffold protein to which Int-C and Int-N are added was purified in accordance with the previously performed purification method using the transduced *E*. *coli.* Nanoperforators each containing Int-N and Int-C domains were produced using purified proteins. After treatment with 2 mM TCEP to increase trans-splicing efficiency, two nanoperforators were mixed. This is because, in the trans-splicing process, it is important that the thiol group, which is a residue of the cysteine amino acid, maintains a free thiol group and does not form a disulfide bond. The result of analysis through size exclusion chromatography (SEC) showed that the double nanoperforator structure formed by linking nanoperforators had about twice the molecular weight of the single nanoperforator. In addition, the result of SDS-PAGE showed that the double nanoperforators separated through SEC have a structure in which two membrane scaffold proteins are linked.

In an embodiment, the lipid contained in the nanoperforator may include a phospholipid, and the membrane scaffold protein may be an amphiphilic protein having a helix structure.

In an embodiment, the surface antigen may be hemagglutinin (HA) or neuraminidase (NA).

In an embodiment, the receptor may be a glycolipid containing sialic acid or a glycoprotein containing sialic acid, and the glycolipid containing sialic acid may be a ganglioside or polysialic acid.

In an embodiment, the molar ratio of the lipid contained in the lipid bilayer nanodisc and/or receptor for the surface antigen, and the membrane scaffold protein may range from 10:1 to 800:1.

In an embodiment, it was found that the double nanoperforator structure exhibited not only inhibitory activity against infection with various kinds of viruses, but also improved antiviral efficacy compared to the antiviral efficacy of conventional nanoperforators against specific influenza virus species. The receptor inserted into the nanoperforator has binding affinity to the hemagglutinin protein of the influenza virus membrane. Such binding affinity affects the activity of inhibiting the influenza virus infection mechanism. Compared to the single nanoperforator structure, the double nanoperforater structure has a higher number of inserted receptors per structure and thus increased binding affinity to the virus. The result of CPE inhibition assay showed that the double nanoperforator had an IC₅₀ of less than twice that of the single nanoperforator at the same receptor concentration. Accordingly, it is considered that the double nanoperforator has increased binding affinity to a virus than the single nanoperforator due to the physical properties thereof.

In another aspect, the present invention is directed to a nanoperforator including the antibody-binding membrane scaffold protein (FcBP-MSP).

In an embodiment, the nanoperforator may include an antibody-binding end-spliced membrane scaffold protein in which the N-terminal domain of the intein is fused to the C-terminus of the fusion protein of the Fc region-binding peptide and the membrane scaffold protein, and the C-terminus of the intein is fused to the N-terminus of the fusion protein to join the N-terminal domain of the fusion protein to the C-terminal domain of the fusion protein to thereby form a cycle.

In an embodiment, the antibody-binding end-spliced membrane scaffold protein may be produced by joininig both termini of the fusion protein in which the Fc region-binding peptide is fused to the membrane scaffold protein using sortase.

In an embodiment, the nanoperforator of the present invention may be pegylated to improve the stability of the construct, resistance to a degrading enzyme, and the biostability.

In one embodiment, the nanoperforator of the present invention may be bound with an antibody or mini-antibody.

In an embodiment, the nanoperforator of the present invention may include a lipid bilayer nanodisc and a membrane scaffold protein surrounding the outer circumferential surface of the lipid bilayer nanodisc, and may further include a receptor for a surface antigen of a virus.

In an embodiment, the nanoperforator is capable of perforating the lipid bilayer envelope of the virus.

In an embodiment, the virus may be a virus having a lipid bilayer envelope (or membrane), and may include at least one selected from the group consisting of the families *Coronaviridae, Bunyaviridae, Filoviridae, Flaviviridae, Hepadnaviridae, Herpesviridae, Orthomyxoviridae, Poxviridae, Rhabdoviridae, Retroviridae* and *Togaviridae,* more preferably an influenza virus.

In another aspect, the present invention is directed to a nanoperforator including the antibody-binding end-spliced large membrane scaffold protein (Fc-eslMSP) according to the present invention.

In an embodiment, the antibody-binding end-spliced large membrane scaffold protein may include a fused protein in which an end-spliced large membrane scaffold protein including two or more membrane scaffold proteins that are fused and linked is fused to an FC-region binding peptide, and the nanoperforator including the same may be a nanoperforator (or end-spliced large ND, eslND) having an increased surface area of 15 nm or more in diameter.

In an embodiment, the antibody-binding end-spliced large membrane scaffold protein (FcBP-eslMSP) may be produced by joining both termini of the fusion protein in which the Fc region-binding peptide is fused to two or more membrane scaffold proteins using sortase.

In an embodiment, the nanoperforator of the present invention may be pegylated to improve the stability of the construct, resistance to a degrading enzyme, and biostability.

In one embodiment, the nanoperforator of the present invention may be bound with an antibody or mini-antibody.

In an embodiment, the nanoperforator of the present invention may include a lipid bilayer nanodisc and a membrane scaffold protein surrounding the outer circumferential surface of the lipid bilayer nanodisc, and may further include a receptor for a surface antigen of a virus.

In an embodiment, it was found that when two or more membrane scaffold proteins are linked in a cyclic structure to thereby increase the area of the nanoperforator, the ability to damage the virus envelope is improved, the particle sizes of the nanoperforators using the membrane scaffold protein are uniform, and the antiviral effect such as an effect of preventing infection of a virus having a lipid bilayer envelope or an effect of inhibiting the proliferation of the infecting virus, is improved.

In the present invention, it was found that the end-spliced large nanoperforator having an increased size due to linking of two or more membrane scaffold proteins facilitated the perforation effect through fusion with the viral membrane, thereby improving the antiviral effect. A membrane scaffold protein gene was inserted into the plasmid for the end-spliced nanoperforator and was produced using an overlap cloning method, but is not limited thereto. In addition, various membrane scaffold proteins are applicable, without limitation to the MSP1E3D1 protein used in the present invention.

In one embodiment, the nanoperforator may have a diameter of 10 to 100 nm, more preferably 15 to 50 nm, but the size of the nanoperforator is not limited, so long as the function of the nanoperforator containing a membrane scaffold protein using a trans-intein can be realized.

In an embodiment, it was found that the nanoperforator using the membrane scaffold protein in which both termini of the amino terminus (N-terminus) and the carboxyl terminus (C-terminus) of the fusion protein, in which the Fc region-binding peptide is fused to the membrane scaffold protein, constituting the nanoperforator are linked through a covalent bond to form a cyclic structure is better able to resist structural damage due to heat and aggregation at high concentrations than the conventional nanoperforator using the linear membrane scaffold protein. The method for joining both termini of a protein to form a cyclic structure may also include other protein-engineering techniques, including a method using trans-intein splicing.

In the present invention, the nanoperforator containing the fusion protein, in which the Fc region-binding peptide is fused to the membrane scaffold protein, can improve the antiviral efficacy thereof. The two termini of the antibody-binding end-spliced membrane scaffold protein constituting the nanoperforator are joined to each other, so the nanoperforator is more resistant to heat, and is more stable and resistant to aggregation at a constant size and high concentration than conventional nanoperforators, so degradation or aggregation due to the temperature of the biological subject receiving treatment and due to proteases is reduced, and antiviral efficacy is improved compared to conventional nanoperforators. In addition, it was found that the end-spliced membrane scaffold protein using the trans-intein had a lower endotoxin level than the conventional linear membrane scaffold protein and apolipoprotein, in particular 1/10 of that of apolipoprotein. This can remarkably reduce the possibility of contamination of the end-spliced membrane scaffold protein using the end-spliced nanoperforator with endotoxins, which cause side effects of high fever, headache, and sepsis (FIG. 3).

In an embodiment, when PEG is attached to the nanoperforator of the present invention, the thermal stability of the nanoperforator is remarkably improved, and at the same time, resistance to protease (e.g., trypsin) is greatly increased. Proteases such as trypsin destroy the structure of the nanoperforator and thus loses the antiviral activity thereof. The PEG-attached nanoperforator, having resistance thereto, has excellent antiviral activity.

In another aspect, the present invention is directed to a double nanoperforator including the antibody-binding double end-spliced membrane scaffold protein (FcBP-esdMSP) according to the present invention.

In an embodiment, the double nanoperforator may have a structure in which two nanoperforators are linked through a disulfide bond.

In an embodiment, the double nanoperforator of the present invention may be pegylated to improve the stability of the construct and thus resistance to a degrading enzyme.

In an embodiment, the double nanoperforator of the present invention may be bound with an antibody or mini-antibody and the two nanoperforators may be bound with antibodies or mini-antibodies of the same type or different types.

The FcBP included in the fusion protein of the present invention binds with high selectivity and specificity to the Fc region of the antibody to be bound. In addition, the FcBP binds to the Fc region rather than the antigen-binding region of the antibody and thus does not affect the antigen-binding affinity of the antibody. In addition, the FcBP may be immobilized on a solid phase, such as a common resin, as an antibody-affinity ligand.

In an embodiment, a protein domain required for linking to the N-terminus of the membrane scaffold protein was added along with a linker in order to bind the nanoperforator to an antibody. Among them, the protein domain includes, but is not limited to, Fcbp (Fc region-binding peptide) set forth in Table 2 below. The Fcbp may bind to an antibody without further chemical or biological treatment after purification. The linker located after the Fcbp functions to provide an effective distance and flexibility so that the nanoperforator can effectively access the virus upon binding between the antibody and the nanoperforator.

**[Table 2]**

| **Name** | **Sequence** |
|---|---|
| **Fc-3** | **TCWVLEGLHWACD** |
| **Fc-3-4c** | **CTCWVlEGLHWACDC** |
| **TWKTSRISIF** | **TWKTSRISIF** |
| **FGRLVSSIRY** | **FGRLVSSIRY** |
| **EPIHRSTLTALL** | **EPIHRSTLTALL** |
| **HWRGWV** | **HWRGWV** |
| **HYFKFD** | **HYFKFD** |
| **HFRRHL** | **HFRRHL** |
| **HWCitGWV** | **HWCitGWV** |
| **NKFRGKYK** | **NKFRGKYK** |
| **NARKFYKG** | **NARKFYKG** |
| **FYWHCLDE (1)** | **FYWHCLDE** |
| **FYCHWALE (2)** | **FYCHWALE** |
| **FYCHTIDE (3)** | **FYCHTIDE** |
| **KHRFNKD** | **KHRFNKD** |
| **RRGW** | **RRGW** |

As used herein, the term "antibody", which is well-known in the art, refers to a specific protein molecule directed toward an antigenic site.

The antibody may be produced using a well-known method. The antibody also includes a peptide fragment that may be produced from the protein. The form of the antibody of the present invention is not particularly limited, and the antibody may include a polyclonal antibody, a monoclonal antibody, a fragment thereof that has antigen-binding affinity, and all immunoglobulin antibodies. Furthermore, the antibody of the present invention includes a specific antibody such as a humanized antibody.

In an embodiment, the double nanoperforator of the present invention may include a lipid bilayer nanodisc and a membrane scaffold protein surrounding the outer circumferential surface of the lipid bilayer nanodisc, and may further include a receptor for a surface antigen of a virus, and two nanoperforators may include different receptors for virus surface antigens.

In another aspect, the present invention is directed to a double nanoperforator including two nanoperforators that are linked through a covalent bond, wherein the double nanoperforator has a structure in which one nanoperforator containing the fusion protein of the Fc region binding peptide and the membrane scaffold protein according to the present invention is linked through a covalent bond to the other nanoperforator containing the fusion protein of the Fc region binding peptide and the membrane scaffold protein according to the present invention.

In an embodiment, the two nanoperforators may be linked through a covalent bond when the intein formed by linking the N-terminal domain of the intein to the C-terminal domain of the intein in the two nanoperforators is removed.

In one embodiment, the membrane scaffold protein of each of the two nanoperforators may include a membrane scaffold protein in which two or more membrane scaffold proteins are repeatedly linked.

In an embodiment, the double nanoperforator may be prepared using a method such as a disulfide bond, a thioether bond, a sortase bond, an intein bond, or the like.

In one embodiment, the double nanoperforator of the present invention may be bound with an antibody or mini-antibody and the two nanoperforators may be bound with antibodies or mini-antibodies of the same type or different types.

In an embodiment, the double nanoperforator of the present invention may be pegylated to improve the stability of the construct and thus resistance to a degrading enzyme.

In an embodiment, the double nanoperforator of the present invention may include a lipid bilayer nanodisc and a membrane scaffold protein surrounding the outer circumferential surface of the lipid bilayer nanodisc, and may further include a receptor for a surface antigen of a virus, and the two nanoperforators may include different receptors for virus surface antigens.

In an embodiment, the lipid contained in the nanoperforator may include a phospholipid, and the membrane scaffold protein may be an amphiphilic protein having a helix structure.

In an embodiment, the surface antigen may be hemagglutinin (HA) or neuraminidase (NA).

In an embodiment, the receptor may be a glycolipid containing sialic acid or a glycoprotein containing sialic acid, and the glycolipid containing sialic acid may be a ganglioside or polysialic acid.

In an embodiment, the molar ratio of the lipid contained in the lipid bilayer nanodisc and/or receptor for the surface antigen, to the membrane scaffold protein may range from 10:1 to 800:1.

As used herein, the term "nanoperforator (NP)" refers to a nanoscale material which includes a lipid bilayer nanodisc and a membrane scaffold protein surrounding the outer circumferential surface of the lipid bilayer, and is capable of performing the function of perforating a viral envelope. The nanoperforator includes a unilamellar lipid bilayer having a disc shape, that is, a lipid bilayer nanodisc, and the outer circumference surface of the lipid bilayer may be a complex surrounded by two or more membrane scaffold proteins, for example, two membrane scaffold proteins. As used herein, the term "lipid bilayer nanodisc" refers to a unilamellar disc-shaped material that contains a lipid bilayer and has an open system in which both surfaces of the lipid bilayer are exposed to the outside. A membrane scaffold protein is a modified form of apolipoprotein A1 (ApoA-1), which is a major component of high-density lipoprotein, and the hydrophobic surface faces lipids and is exposed to the outside, thereby realizing a stable disc shape. Nanodiscs having various sizes may be produced depending on the number of helixes of the membrane scaffold protein, and mainly used membrane scaffold proteins include MSP1D1, MSP1D1dH5, MSP1E3D1, MSP2N2, and the like. The nanodisc can stably dissolve lipids in an aqueous solution without the aid of other surfactants, and has an advantage in which membrane proteins are inserted into the lipid bilayer, facilitating research on membrane proteins even in aqueous solutions. The lipid may, for example, include at least one selected from the group consisting of phosphatidylcholine, phosphatidylglycerol, phosphatidylethanolamine, phosphatidylserine and cholesterol. Any lipid may be used without limitation, so long as it is capable of constituting a bilayer. The phosphatidylcholine may be DOPC (1,2-dioleoyl-sn-glycero-3-phosphocholine), DLPC (1,2-dilauroyl-sn-glycero-3-phosphocholine), DMPC (1,2-dimyristoyl-sn-glycero-3-phosphocholine), DPPC (1,2-dipalmitoyl-sn-glycero-3-phosphocholine), POPC (1-palmitoyl-2-oleoyl-sn-glycero-3-phosphocholine), C13PC, DDPC (1,2-didecanoyl-sn-glycero-3-phosphocholine), DSPC (1,2-distearoyl-sn-glycero-3-phosphocholine), DEPC (1,2-dierucoyl-sn-glycero-3-phosphocholine), DLOPC (1,2-dilinoleoyl-sn-glycero-3-phosphocholine), EPC (egg phosphatidylcholine), MSPC (1-myristoyl-2-stearoyl-sn-glycero-3-phosphocholine), PMPC (1-palmitoyl-2-myristoyl-sn-glycero-3-phosphocholine), PSPC (1-palmitoyl-2-strearoyl-sn-glycero-3-phosphocholine), SMPC (1-strearoyl-2-nyristoyl-sn-glycero-3-phosphocholine) or SPPC (1-strearoyl-2-palmitoyl-sn-glycero-3-phosphocholine), the phosphatidylglycerol may be DMPG (1,2-dimyristoyl-sn-glycero-3[phospho-rac-(1-glycerol)]), DPPG (1,2-dipalmitoyl-sn-glycero-3[phospho-rac-(1-glycerol)]), DSPG (1,2-distearoyl-sn-glycero-3[phospho-rac-(1-glycerol)]), P0PG (1-palmitoyl-2-oleoyl-sn-glycero-3[phospho-rac-(1-glycerol)]), DEPG (1,2-dierucoyl-sn-glycero-3[phospho-rac-(1-glycerol)]), DLPG (1,2-dilauroyl-sn-glycero-3[phospho-rac-(1-glycerol)]), DOPG (1,2-dioleoyl-sn-glycero-3[phospho-rac-(1-glycerol)]) or DSPG (1,2-distearoyl-sn-glycero-3[phospho-rac-(1-glycerol)]), the phosphatidylethanolamine may be DMPE (1,2-dimyristoyl-sn-glycero-3-phosphoethanolamine), DPPE (1,2-dipalmitoyl-sn-glycerol-3-phosphoethanolamine), DSPE (1,2-distearoyl-sn-glycero-3-phosphoethanolamine), DOPE (1,2-dioleoyl-sn-glycero-3-phosphoethanolamine), DEPE (1,2-dierucoyl-sn-glycero-3-phosphoethanolamine), DLPE (1,2-dilauroyl-sn-glycero-3-phosphoethanolamine) or POPE (1-palmitoyl-2-oleoyl-sn-glycero-3-phosphoethanolamine), and the phosphatidylserine may be DOPS (1,2-diol eoyl-sn-glycero-3-phosphoserine), DLPS (1,2-dilauroyl-sn-glycero-3-phosphoserine), DMPS (1,2-dimyristoyl-sn-glycero-3-phosphoserine), DPPS (1,2-dipalmitoyl-sn-glycero-3-phosphoserine), DSPS (1,2-distearoyl-sn-glycero-3-phosphoserine) or POPS.

In addition to the nanodisc, the nanoperforator according to the present invention may further include a receptor for a surface antigen of a virus having an envelope of the lipid bilayer. The nanoperforator of the present invention may include one or more receptors.

In one embodiment of the present invention, the nanoperforator including the membrane scaffold protein surrounding the lipid bilayer is also referred to as a nanodisc. That is, in the present invention, the term "nanoperforator (NP)" is used interchangeably with "nanodisc (ND)". However, the term "nanodisc" is intended to be used to refer to the structural aspect of phospholipid nanoparticles, whereas the term "nanoperforator" is intended to be used to refer to the functional aspect of antiviral activity. In addition, in the experiment to compare the case in which a receptor is included, with the case in which no receptor is included, the case in which a receptor is not included is referred to as "NP" and the case, in which a receptor (e.g. TG (total ganglioside) of a ganglioside family that mainly includes GD1a, GT1b and GM1) is included, is referred to as "NPTG (or NDTG)". However, in the absence of such a distinction, only structural characteristics are emphasized.

In one embodiment, in addition to the phospholipid, the lipid constituting the lipid bilayer nanodisc, may include one or more selected from the group consisting of neutral fat (for example, triglycerides), cholesterol or a derivative thereof, and saccharolipids (for example, gangliosides).

As used herein, the term "virus having a lipid bilayer envelope (or membrane)" refers to a type of virus having a lipid bilayer envelope, wherein the antigen protein involved in infection and proliferation of the virus is contained in a membrane-bound protein form in the lipid bilayer. The virus having the lipid bilayer envelope is not particularly limited, as long as the nanoperforator provided in the present invention exhibits antiviral activity, and may be, for example, a virus belonging to any of the families *Coronaviridae, Bunyaviridae, Filoviridae, Flaviviridae, Hepadnaviridae, Herpesviridae, Orthomyxoviridae, Poxviridae, Rhabdoviridae, Retroviridae* and *Togaviridae.* Other examples of the virus include: Sin Nombre hantavirus, etc., which belongs to the *Bunyaviridae* family; coronavirus, etc., involved in various acute respiratory syndromes, which belongs to the *Coronaviridae* family; Ebola virus, Marburg virus, etc., which belong to the *Filoviridae* family; West Nile virus, yellow fever virus, Dengue fever virus, Hepatitis C virus, etc., which belong to the *Flaviviridae* family; hepatitis B virus, etc., which belongs to the *Hepadnaviridae* family; herpes simplex 1 virus, herpes simplex 2 virus, etc., which belong to the *Hepadnaviridae* family; influenza virus etc., which belongs to the *Orthomyxoviridae* family; smallpox virus, vaccinia virus, molluscum contagiosum virus, monkeypox virus, etc., which belong to the *Poxviridae* family; rabies virus, etc., which belongs to the *Rhabdoviridae* family; HIV (human immunodeficiency virus) etc., which belongs to the *Retroviridae* family; Chikungunya virus etc., which belongs to the *Togaviridae* family; and pseudorabies virus, HHV virus, etc., which belong to the *Herpesviridae* family. Another example thereof may be an influenza virus belonging to *Orthomyxoviridae.* For example, the virus may be a virus having affinity for a receptor (e.g., ganglioside) included in the nanoperforator.

As used herein, the term "influenza virus" refers to an RNA virus that belongs to *Orthomyxoviride,* and is divided into three serotypes, namely, type A, type B, and type C. The serotypes of influenza A virus are classified depending on the type of two proteins, i.e., hemagglutinin (HA) and neuraminidase (NA), on the surface of the virus. 144 types of influenza A virus (16 types of HA proteins and 9 types of NA proteins) are known to date.

As used herein, the term "surface antigen", which is interchangeable with "cell membrane antigen", refers to an antigenic membrane-bound protein present in the cell membrane of cells. In the present invention, the surface antigen may be interpreted as meaning a membrane-bound protein bound to a lipid bilayer of a virus having an envelope of the lipid bilayer, and the surface antigen is, for example, hemagglutinin (HA), neuraminidase (NA), or the like, which is a surface antigen of an influenza virus, but is not particularly limited thereto. As used herein, the term "hemagglutinin (HA)" is a transmembrane protein that is a type of influenza virus surface antigen, and is composed of an HA1 subunit and an HA2 subunit that may be cleaved by trypsin. It is known that the HA1 subunit binds to sialic acid, whereas the HA2 subunit induces cell membrane fusion under low pH conditions.

As used herein, the term "receptor for surface antigen" refers to a receptor capable of binding to the surface antigen, and may be an antibody against the surface antigen, or may be another cell membrane-binding protein to which the surface antigen is capable of binding.

In the present invention, the receptor for the surface antigen may be interpreted as meaning a receptor that is present on the surface of a host cell that may be infected with a virus having a lipid bilayer envelope and is capable of binding to a surface antigen of the virus. The receptor and the surface antigen of the virus may be bound by various interactions such as a hydrogen bond or an ionic bond. For example, sialic acid may be bound to the receptor-binding site of the outermost surface of the HA1 subunit of hemagglutinin of the virus. Accordingly, the receptor of the present invention may be a receptor capable of forming a specific or affinity binding to a target virus, for example, a virus containing hemagglutinin or neuraminidase.

In one embodiment, the type of receptor for the surface antigen may include sialic acid or a sialic acid-like functional group (e.g., a peptide mimicking sialic acid), but is not particularly limited thereto. The receptor may include at least one selected from the group consisting of sialyloligosaccharide (for example, ganglioside), glycoprotein, and polysialic acid. However, any receptor may be used without limitation as long as it contains sialic acid.

In an embodiment, the functional group may be incorporated in or bound to the lipid bilayer by the receptor itself, or may be incorporated in or bound to the lipid bilayer through a linker.

In an embodiment, the receptor may be bound to the lipid of the nanodisc lipid bilayer by the receptor itself or the linker bound with the receptor through various interactions such as a hydrogen bond, ionic bond, covalent bond, disulfide bond, or the like.

In an embodiment, an example of the receptor is sialic acid present in the cell membrane of respiratory cells capable of binding to hemagglutinin, which is an influenza virus surface antigen. For example, the receptor contains the sialic acid and ganglioside binding to the cell membrane.

In the present invention, the term "ganglioside" refers to a compound in which at least one sialic acid is bound to a sugar chain of glycosphingolipid through a specific bond (α-2,3 linkage or α-2,6 linkage), and may include ganglioside containing α-2,3-linked sialic acid and ganglioside containing α-2,6-linked sialic acid. For example, the ganglioside of the present invention includes GM1, GM2, or GM3 containing one N-acetylneuraminic acid or sialic acid, GD1a, GD1b, GD2, or GD3 containing two N-acetylneuraminic acids, GTlb or GT3 containing three N-acetylneuraminic acids, or GQ1 containing four N-acetylneuraminic acids.

In the present invention, among gangliosides having α-2,6 linkage, GDla has two N-acetylneuraminic acids (sialic acids). The result of plaque formation inhibition assay and CPE inhibition assay showed that the nanoperforator having GDla exhibited strong antiviral efficacy against A/PR/8/34, A/Sydney/5/97, and A/aquatic bird/Korea/w81/2005 among influenza virus strains. However, the nanoperforator having GDla exhibited weak antiviral efficacy against the A/X-31 strain. In addition, the receptor (2,6SL), which has an artificial α-2,6 sialic acid form, exhibits strong selective antiviral efficacy against the influenza virus A/X-31 strain, but exhibits weak infection inhibitory effect against the three strains of influenza virus against which the nanoperforator having GDla exhibited a weak antiviral efficacy. When a combination of a single nanoperforator having GDla with a single nanoperforator having a 2,6SL receptor was used in order to overcome this problem, antiviral efficacy against 4 strains was detected, but the efficacy thereof was remarkably reduced compared to the case in which the single nanoperforator was used alone at the same concentration. It was found that when two other receptors, namely, GDla and 2,6SL, were inserted into one nanoperforator, only the efficacy of the 2,6SL receptor, which could be more exposed to the outside, was observed.

In an embodiment, the receptor, for example, ganglioside, is inserted into the lipid bilayer nanodisc region of the lipid bilayer nanoperforator, and functions to bind to the HA of the virus having the lipid bilayer envelope.

The nanoperforators provided in the present invention have the following characteristics:

End-spliced nanoperforator (essND or esdND): this is a nanoperforator including an end-spliced membrane scaffold protein (esMSP) having two termini joined to each other, in which the C-terminal domain of a Cfa intein is linked to one terminus of the membrane scaffold protein and the N-terminal domain of the Cfa intein is linked to the other terminus of the membrane scaffold protein, and may be produced as an end-spliced single nanoperforator (essND) (treated with a reducing agent) or an end-spliced double nanoperforator (esdND) (not-treated with a reducing agent) containing an end-spliced double membrane scaffold protein linked by a disulfide bond depending on whether or not treatment with a reducing agent is performed during esMSP purification;

End-spliced large nanoperforator (eslND): a nanoperforator containing a protein (eslMSP) that has an increased size based on the length of a double helix of esMSP (two repeating MSPs);

Double nanoperforator (Di-NP): a double nanoperforator obtained by producing nanoperforators using membrane scaffold proteins attached with CfaN and CfaC inteins (CfaC-MSP and MSP-CfaN), and covalently bonding the nanoperforators through trans-splicing;

Antibody-binding end-spliced nanoperforator (Fc-essND or Fc-esdND): a nanoperforator containing an end-spliced fused protein (Fc-esMSP, antibody-binding end-spliced membrane scaffold protein) having two termini joined to each other in which the C-terminal domain of a Cfa intein is linked to one terminus of a fusion protein of an Fc region-binding peptide and a membrane scaffold protein and the N-terminal domain of the Cfa intein is linked to the other terminus of the fusion protein, and may be produced as an end-spliced single nanoperforator (Fc-essND) (treated with a reducing agent) or an end-spliced double nanoperforator (Fc-esdND) (not-treated with a reducing agent) containing an end-spliced double fusion protein linked by a disulfide bond, depending on whether or not treatment with a reducing agent is performed during Fc-esMSP purification;

Antibody-binding end-spliced large nanoperforator (Fc-eslND): a nanoperforator containing a protein (Fc-eslMSP) that has an increased size based on the length of a double helix of esMSP (two repeating MSPs); and

Antibody-binding double nanoperforator (Di-NP): double nanoperforator obtained by producing nanoperforators using a fusion protein of an Fc region-binding peptide and a membrane scaffold protein, attached with CfaN and CfaC inteins (CfaC-FcMSP and MSP-FcCfaN), and covalently bonding the nanoperforators through trans-splicing.

The nanoperforators may be derived from the structure of the pegylated nanoperforators to which BM(PEG)₃ is attached.

In another aspect, the present invention is directed to a pharmaceutical composition for preventing or treating a viral infection containing the nanoperforator of the present invention.

In an embodiment, the virus may include at least one selected from the group consisting of the families *Coronaviridae, Bunyaviridae, Filoviridae, Flaviviridae, Hepadnaviridae, Herpesviridae, Orthomyxoviridae, Poxviridae, Rhabdoviridae, Retroviridae* and *Togaviridae,* but is not limited thereto.

In one embodiment, it has been found that when the nanoperforator of the present invention is attached to an antibody, the efficacy of the antibody can be dramatically improved. In this case, the antibody capable of binding to the nanoperforator may target a protein constituting the virus, such as hemagglutinin, neuraminidase, and a M2 ion channel protein, which are influenza virus surface antigens. In addition, other proteins or peptides that bind to the above surface antigens may be used instead of the antibody to improve the structure of the membrane scaffold protein.

In one embodiment, the complex including each of the constructs shown in (A), (B), (C) and (D) of FIG. 28 below may be used as a composition for treating and/or ameliorating antiviral conditions. In one embodiment, it was found that the effect of the antiviral antibody can be maximized by introducing a protein sequence capable of binding to the Fc portion of the antibody into the nanoperforator, and that a construct, in which an antibody-binding nanoperforator (FcND) is bound to an antibody, exhibits further improved antiviral antibody, compared to a single antibody or a combination of an antibody with a nanoperforator. It was found that when the above invention is introduced into a broad neutralizing antibody (bnAb), the antiviral effect of the antibody against all influenza virus strains that bnAb can inhibit can be improved.

The nanoperforator of the present invention, for example, a nanoperforator containing ganglioside as a surface antigen receptor can disrupt the intracellular infection pathway of viruses infecting host cells, for example, an influenza virus, thereby inhibiting proliferation of the virus.

In general, the HA1 subunit constituting the HA of the virus binds to sialic acid in the host cell membrane and invades the host cells through endocytosis. Meanwhile, when a virus-infected cell is treated with the nanoperforator of the present invention, for example, a nanoperforator containing a ganglioside as a receptor, the HA of the virus can bind not only to the host cell membrane, but also to the lipid bilayer nanodisc and/or the receptor of the nanoperforator. When the amount of nanoperforator that is treated is increased, the proportion of the virus that is bound to the nanoperforator is increased, thereby preventing the virus from infecting the host cells. Therefore, the nanoperforator may be an entry inhibitor that suppresses endocytosis (intracellular invasion) of the virus using the receptor of the host cell as a mimic "decoy" (primary inhibition).

In the membrane fusion step induced in the late endosomal stage during endocytosis (intracellular invasion) of the virus, the virus bound to the nanoperforator of the present invention does not undergo membrane fusion between the viral envelope and the cell membrane of the host cell, but undergoes membrane fusion between the virus envelope and the lipid bilayer of the nanoperforator. Such cellular membrane fusion occurs probabilistically. When one host cell membrane and a plurality of nanoperforators bind to one virus, membrane fusion occurs between the virus envelope and the lipid bilayer of the nanoperforator, rather than between the virus envelope and the cell membrane of the host cell. As such, when membrane fusion is induced between the virus envelope and the lipid bilayer of the nanoperforator in the endosome, the RNA present inside the virus is released into the endosome through the membrane fusion site and the released RNA is inactivated due to the low pH in the endosome, ultimately resulting in degradation. Therefore, the nanoperforator of the present invention may be a perforator that perforates the viral envelope in the membrane fusion stage of the viral endosomal stage (secondary inhibition).

That is, the nanoperforator binds to the virus envelope and inhibits the intracellular invasion of the virus to form endosomes (primary inhibition), and even if the virus invades a cell, the nanoperforator acts as a "perforator" that perforates the envelope of the virus that has invaded the cell (secondary inhibition) and thus has an effect of secondarily inhibiting viral infection. Therefore, when cells infected with the virus having a lipid bilayer envelope are treated with a nanoperforator containing a surface antigen receptor, for example, a ganglioside, the nanoperforator blocks the infection pathway of the virus, thereby inhibiting infection by the virus, ultimately inhibiting proliferation of the virus, and exhibiting an effect of treating diseases caused by viral infection.

In summary, the nanoperforator provided in the present invention inhibits infection of host cells with the virus having a lipid bilayer envelope, or fundamentally suppresses proliferation after infection, thereby exhibiting an effect of preventing or treating diseases caused by viral infection. Accordingly, when the lipid bilayer nanoperforator containing ganglioside provided in the present invention can exhibit the identical effect against a virus capable of binding to host cells via sialic acid of the host cell, regardless of whether the virus is modified (mutated) or not, can suppress initial infection of virus, and has an advantage of fundamentally inhibiting proliferation of the virus, even after the host is infected with the virus.

Therefore, the nanoperforator of the present invention is not dependent on viral mutation. In particular, the nanoperforator has an advantage of ensuring safety because it does not contain a material that induces a specific reaction *in vivo.*

In one embodiment, the nanoperforator of the present invention may be bound with at least one marker selected from the group consisting of histidine (His), gold (Au), fluorescent lipids, biotin/avidin, and fluorescent materials.

In one embodiment, the molar ratio of at least one selected from the group consisting of the lipid constituting the lipid bilayer and surface antigen receptor included in the nanoperforator to the membrane scaffold protein ([(number of moles of lipid bilayer nanodisc lipid)) + (number of moles of the receptor for the surface antigen)]: number of moles of the membrane scaffold protein) is 10:1 to 800:1, preferably 50:1 to 500:1, more preferably 50:1 to 150:1, for example, 65:1 or 125:1. For example, the molar ratio may be the molar ratio of the lipid of the nanodisc to the membrane scaffold protein, or the molar ratio of the sum of the moles of the lipid of the nanodisc and the moles of the surface antigen receptor to the membrane scaffold protein.

In one embodiment, the surface antigen receptor, for example, ganglioside, contained in the nanoperforator of the present invention is present in an amount of 0.01 to 99 mol%, preferably 1 to 90 mol%, more preferably 15 mol% or more, or 10 to 50 mol%, based on 100% of the total number of moles of the nanodisc (e.g., the sum of the number of moles of the lipid and the number of moles of the receptor).

The nanoperforators provided in the present invention may be expected to have improved effects of preventing and treating viral infection compared to conventional nanoperforators through modification of the properties of the membrane scaffold protein.

In one embodiment, the nanoperforator of the present invention may be applied to a pharmaceutical composition for treating, preventing or ameliorating one or more symptoms associated with or resulting from a viral infection or delaying the onset of one or more symptoms associated with or resulting from the viral infection, and to use for treatment, prevention, or amelioration of the symptoms, or delaying the onset of symptoms.

The nanoperforator of the present invention can disrupt the infection pathway of a virus having a lipid bilayer envelope or inhibit the proliferation of viral infection, thus being used to prevent or treat various infections caused by infection with a virus having a lipid bilayer envelope. The composition for preventing or treating viral infection according to the present invention can be applied independently to viral mutations, and the nanoperforator has an advantage of ensuring safety because it does not contain a substance that induces a specific reaction *in vivo.*

As used herein, the term "viral infection" refers to a disease caused by infection with a virus having a lipid bilayer envelope. Examples of the viral infection include: hemorrhagic fever with renal syndrome (epidemic hemorrhagic fever) caused by infection with a virus of the family *Verniaviridae;* respiratory diseases such as coryza caused by infection with a virus of the family *Coronaviridae;* hepatitis C caused by infection with a virus of the family *Flaviviridae;* hepatitis B caused by infection with a virus of the family *Hepadnaviridae;* herpes zoster caused by infection with a virus of the family *Herpesviridae;* flu or influenza virus infection caused by infection with a virus of the family *Orthomyxoviridae;* smallpox caused by infection with a virus of the family *Poxviridae;* rabies or bullous stomatitis caused by infection with a virus of the family *Rhabdoviridae;* acquired immunodeficiency syndrome, etc. caused by infection with a virus of the family *Retroviridae;* and the like. As another example, the viral infection may be flu or influenza viral infection caused by infection with an influenza virus that belongs to the family *Orthomyxoviridae.*

The nanoperforator contained in the composition of the present invention may include one or more surface antigen receptors. For example, two or more receptors may be contained in one nanoperforator. The composition may also contain two or more nanoperforators including one or more different receptors.

As used herein, the term "treatment" refers to activity of improving or positively changing the symptoms of an infectious disease caused by a viral infection. Those skilled in the art to which the present invention pertains can appreciate exact criteria of diseases against which the composition of the present application is effective, and determine the extent of amelioration, improvement, and treatment with reference to data provided by the Korean Medical Association, etc.

As used herein, the term "prevention" refers to prevention of the onset, recurrence, or transmission of a disease or disorder, or one or more symptoms caused by the disease or disorder, and includes prophylactic treatment for potential candidates.

The composition of the present invention may be prepared in the form of a pharmaceutical composition for preventing or treating inflammatory diseases further comprising an appropriate carrier, excipient, or diluent commonly used in the preparation of pharmaceutical compositions, and the carrier may be a natural carrier. Specifically, the pharmaceutical composition may be prepared into an oral formulation such as a powder, granule, tablet, capsule, suspension, emulsion, syrup, or aerosol, or a formulation such as an external preparation, suppository, or a sterile injection solution in accordance with a conventional method. In the present invention, the pharmaceutical composition may contain at least one from the group consisting of various carriers, excipients and diluents that may be contained in the pharmaceutical composition.

The content of the nanoperforator in the pharmaceutical composition of the present invention is, for example, 0.0001 to 10% by weight, or 0.01 to 3% by weight, based on the total weight of the final composition, but is not limited thereto.

The therapeutically effective amount of the composition of the present invention may vary depending on several factors, for example, the administration method, the target site, the condition of the patient, and the like. Therefore, when used in the human body, the dosage should be determined as an appropriate amount in consideration of both safety and efficacy. The amount for use in humans may be estimated based on effective amounts determined through animal experimentation. Considerations for determining the effective amount are described, for example, in Hardman and Limbird, eds., Goodman and Gilman's The Pharmacological Basis of Therapeutics, 10th ed. (2001), Pergamon Press; and E.W. Martin ed., Remington's Pharmaceutical Sciences, 18th ed.(1990), Mack Publishing Co.

The pharmaceutical composition of the present invention may be administered in a pharmaceutically effective amount. As used herein, the term "pharmaceutically effective amount" means an amount sufficient to treat or prevent a disease at a reasonable benefit/risk ratio applicable to pharmaceutical (medical) treatment, while causing no side effects. The effective dosage is determined depending on factors including the state of health of the patient, the disease type, severity, drug activity, drug sensitivity, administration method, administration time, administration route, excretion rate, treatment period, drugs used in combination with or concurrently with the composition of the present invention, and other factors well known in the pharmaceutical field. The composition of the present invention may be administered as a single therapeutic agent or in combination with other therapeutic agents, sequentially or simultaneously with conventional therapeutic agents, and in one or multiple doses. Taking into consideration these factors, it is important to administer the composition in the minimum amount sufficient to achieve maximum efficacy without side effects, which can be easily determined by those skilled in the art.

The composition of the present invention may further contain a carrier, diluent, or excipient commonly used in biological agents, or a combination of two or more thereof. Any pharmaceutically acceptable carrier may be used without particular limitation, as long as it is suitable for *in-vivo* delivery of the composition. For example, the pharmaceutically acceptable carrier may be a compound, saline, sterile water, Ringer's solution, buffered saline, dextrose solution, maltodextrin solution, glycerol, ethanol, or a combination of two or more of these components described in Merck Index, 13th ed., Merck & Co. Inc. If necessary, other conventional additives such as antioxidants, buffers, and bacteriostats may be added. In addition, the composition may be prepared into an injectable formulation, such as an aqueous solution, suspension, or emulsion, or a pill, a capsule, a granule, or a tablet by further adding a diluent, dispersant, surfactant, binder, or lubricant. Furthermore, the composition may be preferably formulated according to the corresponding disease or component using an appropriate method in the art or a method disclosed in Remington's Pharmaceutical Science (Mack Publishing Company, Easton PA, 18th, 1990).

As used herein, the term "pharmaceutically acceptable" means a property of being non-toxic to cells or humans exposed to the composition.

The pharmaceutical composition of the present invention may further contain a pharmaceutically acceptable additive. Useful pharmaceutically acceptable additives include starch, gelatinized starch, microcrystalline cellulose, lactose, povidone, colloidal silicon dioxide, calcium hydrogen phosphate, lactose, mannitol, syrup, gum arabic, pregelatinized starch, corn starch, powdered cellulose, hydroxypropyl cellulose, Opadry, sodium starch glycolate, lead carnauba, synthetic aluminum silicate, stearic acid, magnesium stearate, aluminum stearate, calcium stearate, sucrose, dextrose, sorbitol, talc, and the like. The pharmaceutically acceptable additive according to the present invention is preferably present in an amount of 0.1 to 90 parts by weight based on the weight of the composition, but is not limited thereto.

As used herein, the term "administration" means supplying a predetermined substance to a patient using any suitable method, and may be classified into parenteral administration (e.g., using an intravenous, subcutaneous, intraperitoneal, or topical injection formulation) and oral administration depending on the intended method, and the dosage may vary greatly according to the patient's weight, age, gender, state of health, diet, administration time, administration method, excretion rate, and severity of disease.

The composition of the present invention may be administered parenterally (for example, intravenously, subcutaneously, intraperitoneally, or topically) or orally depending on the desired method, and the dosage may be determined in consideration of the age, weight, gender, physical condition and the like of the subject.

The dosage of the pharmaceutical composition of the present invention can be determined by those skilled in the art in consideration of the purpose of use, the degree of addiction of the disease, the age, weight, gender and disease history of the patient, or the type of substance used as an active ingredient. For example, the pharmaceutical composition of the present invention may be administered in an amount from about 0.1 ng to about 100 mg/kg, preferably from 1 ng to about 10 mg/kg per adult. The administration frequency of the composition of the present invention is not particularly limited and the pharmaceutical composition may be administered once a day or several times a day, divided into multiple doses. The dosage does not limit the scope of the present invention in any way.

The administration of the pharmaceutical composition for treating viral infection of the present invention may be performed through any general route as long as it is capable of reaching the target tissue. The administration of the pharmaceutical composition of the present invention may be achieved through intraperitoneal administration, intravenous administration, intramuscular administration, subcutaneous administration, intradermal administration, oral administration, intranasal administration, intrapulmonary administration, rectal administration, or the like, but is not limited thereto. However, when administered orally, the nanoperforator may be denatured or destroyed by gastric acid, so the oral composition should be formulated so as to be coated with an active agent or to protect the nanoperforator from degradation in the stomach. In addition, the composition may be administered by any device capable of delivering the active substance to a target cell.

In another aspect, the present invention is directed to a composition for inhibiting virus proliferation, containing the nanoperforator according to the present invention.

In an embodiment, the viral proliferation may be inhibited in a manner whereby the nanoperforator binds to a viral envelope and perforates the viral envelope.

In another aspect, the present invention is directed to a method of treating viral infection including administering the pharmaceutical composition in a pharmaceutically effective amount to a subject having or likely to develop a viral infection caused by infection with a virus having a lipid bilayer envelope.

As used herein, the term "subject" encompasses mammals including humans, mice, livestock, and the like, farmed fish, and the like which have or are likely to develop a viral infection caused by infection with a virus having a lipid bilayer envelope, without limitation thereto.

In another aspect, the present invention is directed to a method of producing a nanoperforator containing an end-spliced membrane scaffold protein, the method including: expressing and purifying the plasmid according to the present invention to obtain an end-spliced membrane scaffold protein; and producing a nanoperforator through a self-assembly process.

In an embodiment, the method of producing an essND according to the present invention includes: expressing a plasmid including a nucleotide sequence encoding a fusion protein, wherein an N-terminal domain of an intein is fused to a C-terminus of a membrane scaffold protein and a C-terminal domain of the intein is fused to an N-terminus of the membrane scaffold protein according to the present invention; purifying the end-spliced membrane scaffold protein (esMSP) by treatment with a reducing agent; and producing a nanoperforator through a self-assembly process.

In an embodiment, the reducing agent may be TCEP.

In an embodiment, the method of producing an eslND according to the present invention includes:
expressing a plasmid including a nucleotide sequence encoding a fusion protein sequentially including a C-terminal domain of an intein, two or more membrane scaffold proteins, and an N-terminal domain of the intein; purifying an end-spliced large membrane scaffold protein (eslMSP); and producing a nanoperforator through a self-assembly process.

In one embodiment, the plasmid may be constructed to express a fusion protein including two or more repeating membrane scaffold proteins.

In another aspect, the present invention is directed to a method of producing a double nanoperforator.

In an embodiment, the method of producing the double nanoperforator (esdND) of the present invention includes:
expressing a plasmid including a nucleotide sequence encoding a fusion protein, wherein an N-terminal domain of an intein is fused to a C-terminus of a membrane scaffold protein and a C-terminal domain of the intein is fused to an N-terminus of the membrane scaffold protein; purifying an end-spliced double membrane scaffold protein (esdMSP), in which two end-spliced membrane scaffold proteins are linked through a disulfide bond, without using a reducing agent; and producing a nanoperforator through a self-assembly process.

In an embodiment, the method of producing the double nanoperforator (Di-ND) of the present invention includes:
expressing a plasmid including a nucleotide sequence encoding a fusion protein, wherein a C-terminal domain of an intein is fused to an N-terminus of a membrane scaffold protein, followed by purification, to obtain the fusion protein wherein the C-terminal domain of the intein is fused to the N-terminus of the membrane scaffold protein; producing a first nanoperforator through a self-assembly process; expressing a plasmid including a nucleotide sequence encoding a fusion protein, wherein an N-terminal domain of an intein is fused to a C-terminus of a membrane scaffold protein, followed by purification, to obtain a fusion protein wherein the N-terminal domain of the intein is fused to the C-terminus of the membrane scaffold protein; producing a second nanoperforator through a self-assembly process; and covalently bonding the first nanoperforator to the second nanoperforator.

In another aspect, the present invention is directed to a method of producing a nanoperforator, the method including: expressing the plasmid of the present invention, followed by purification, to obtain an antibody-binding fusion protein; and producing a nanoperforator through a self-assembly process.

In an embodiment, the method of producing an Fc-essND according to the present invention includes:
expressing a plasmid including a nucleotide sequence encoding an antibody-binding membrane scaffold protein (FcBP-MSP); purifying the antibody-binding membrane scaffold protein (FcBP-MSP) by treatment with a reducing agent; and producing a nanoperforator through a self-assembly process.

In an embodiment, the reducing agent may be TCEP.

In an embodiment, the method for preparing an Fc-eslND according to the present invention includes:
expressing a plasmid including a nucleotide sequence encoding an antibody-binding large membrane scaffold protein (FcBP-eslMSP); purifying the antibody-binding end-spliced large membrane scaffold protein (Fc-eslMSP) including two or more repeating membrane scaffold proteins; and producing a nanoperforator through a self-assembly process.

In an embodiment, the plasmid may be constructed to express a fusion protein in which an Fc-region binding peptide is fused to two or more repeating membrane scaffold proteins to form a cycle.

In another aspect, the present invention is directed to a method of producing a double nanoperforator.

In an embodiment, the method of producing the Fc-esdND of the present invention includes:
expressing a plasmid including a nucleotide sequence encoding an antibody-binding end-spliced membrane scaffold protein; purifying an antibody-binding end-spliced double membrane scaffold protein (FcBP-esdMSP) including two or more repeating end-spliced membrane scaffold proteins that are linked through a disulfide bond, without a reducing agent; and producing a nanoperforator through a self-assembly process.

In an embodiment, the method of producing an Fc-Di-ND of the present invention includes:
expressing a plasmid including a nucleotide sequence encoding a fusion protein of an Fc region-binding peptide and a membrane scaffold protein, wherein a C-terminal domain of an intein is fused to an N-terminus of the fusion protein, followed by purification, to obtain the fusion protein of the Fc region-binding peptide and the membrane scaffold protein; producing a first nanoperforator through a self-assembly process; expressing a plasmid including a nucleotide sequence encoding a fusion protein of an Fc region-binding peptide and a membrane scaffold protein, wherein an N-terminal domain of an intein is fused to a C-terminus of the fusion protein, followed by purification, to obtain the fusion protein of the Fc region-binding peptide and the membrane scaffold protein; producing a second nanoperforator through a self-assembly process; and covalently bonding the first nanoperforator to the second nanoperforator.

In another aspect, the present invention is directed to the use of the nanoperforator as an antiviral agent.

In another aspect, the present invention is directed to a method of treating a viral infection using the nanoperforator of the present invention.

### [Mode for Invention]

Hereinafter, the present invention will be described in more detail with reference to the following examples, but the scope of the present invention is not limited to the examples, and includes variations and technical concepts equivalent thereto.

### Example 1. Production of nanoperforator esNP containing end-spliced membrane scaffold protein

### 1-1. Production of plasmid for production of end-spliced membrane scaffold protein (esMSP) using Cfa intein

DNA sequence information for CfaN and CfaC was acquired from Stevens A.J., et al., 2016, Design of a Split Intein with Exceptional Protein Splicing Activity, J. Am. Chem. Soc. In addition, the linker sequence of the CfaC-linker used herein was GGGGSGGGGSGGGGS, and a His-tag including 7 repeated histidine sequences was used for the CfaN-His-tag. The MSP1E3D1 gene sequence was arranged between the CfaC-linker and the CfaN-His-tag, so the final gene construct was CfaC-linker-MSP1E3D1-HisX7-CfaN (hereinafter referred to as "esMSP; end-spliced membrane scaffold protein") (FIG. 1). Gene synthesis, cloning, and sequencing were outsourced (Cosmogene Tech, Korea), and the DNA sequence (SEQ ID NO: 1) of the obtained CfaC-linker-MSP1E3D1-HisX7-CfaN (esMSP) is shown in Table 3 below.

**[Table 3]**

| | |
|---|---|
| CfaC-linker-MSP1E3D1-HisX7-CfaN (esMSP) | ggattgaatgcacagtctatacggtagataaaaatggctttgtgtataca caacctattgctcagtggcataaccggggagaacaggaagttttcgaata ctgcttagaagacggttcgattatccgtgcaacgaaagatcacaaattta tgacgaccgacggtcagatgttaccgattgatgagattttcgaacggggg ttagacctgaaacaagttgatggtttgccgtaa-3' |

### 1-2. Purification of esMSP

BL21(DE3) competent cells were transformed with each of the produced plasmids through heat shock for protein purification. Colonies grown on a solid medium containing 50 µg/ml of kanamycin were inoculated into a liquid medium (LB broth miller) containing 10 ml of kanamycin, cultured for 16 hours at 37°C in a shaking incubator, and secondarily inoculated into 600 ml of the same liquid medium. When *E. coli* grew to an OD₆₀₀ of 0.5 to 0.8, protein expression was performed by treatment with 0.5 mM isopropyl β-D-1-thiogalactopyranoside (IPTG). After expression at 37°C and 150 rpm for 3 hours, centrifugation was performed at 6,000 rpm for 10 minutes to remove the medium and extract only cells. The cells were resuspended using tris buffer containing 1% Triton X-100 and were then disrupted using sonication. The cell substrate was isolated through centrifugation and treated with Ni-NTA agarose beads. Substances that could not be bound to the beads were removed using a buffer containing each of Triton X-100, sodium cholate, and low-concentration imidazole, and the protein to be purified was collected using highly concentrated imidazole. For reference, purification was performed using a buffer containing no 2 mM TCEP. In this process, in the Cfa intein trans-splicing process to realize esMSP protein end-splicing, the thiol groups, which are cysteine amino acid residues, reacted with each other due to the high reactivity thereof to form a disulfide bond, so esMSP proteins treated with or not treated with TCEP, R (reducing), and NR (non-reducing) were electrophoresed on SDS-PAGE gel and identified by Coomassie staining. As a result, as shown in the top of FIG. 2, most of the esMSP proteins not treated with TCEP were purified in a form in which two esMSP proteins are linked by a disulfide bond, and this form is freely controllable depending on the presence or absence of TCEP. In addition, the result of treatment of the purified protein with DAPase used to remove the N-terminal His-tag of the protein showed that the control MSP1E3D1 protein was reduced in size due to removal of the N-terminal His-tag by DAPase, whereas esMSP had no size change due to the absence of reaction with DAPase. As a result, the end-spliced structure of esMSP was identified (bottom of FIG. 2).

In addition, LAL analysis was performed in order to measure the amount of endotoxin of esMSP compared to ApoA-1 and MSP1E3D1, which are conventionally used to produce nanodiscs. Three proteins having similar concentrations were diluted 2,000x, 4,000x, and 8,000x, respectively, an endotoxin concentration was measured, an EU (endotoxin unit) per 1 mL was measured using a standard endotoxin with a known endotoxin concentration, and the amount of endotoxin per 1 mg of protein was calculated using the protein concentration. The result of measurement showed that ApoA-1 had the highest endotoxin concentration of 171.64 EU/mg and MSP1E3D1 had an endotoxin concentration of 53.28 EU/mg. However, esMSP had an endotoxin concentration of 24.62 EU/mg, which was lower than that of MSP1E3D1. This demonstrated that, among the membrane scaffold proteins obtained through the purification process under the same conditions, the end-spliced (cyclic) construct, esMSP, exhibited lower binding affinity to endotoxin and had a lower endotoxin concentration than other linear membrane scaffold proteins (bottom of FIG. 3).

### 1-3. Determination of thermal stability of esMSP

Samples were prepared for respective temperatures at which 200 µl of the purified esMSP and the control MSP1E3D1 protein were treated at a concentration of 40 uM. The prepared samples were incubated at the respective temperatures for 30 minutes and centrifuged at 20,000 g for 1 hour to separate the supernatant, and the settled precipitate was sufficiently released with 200 µl of a buffer (100 mM NaCl, 40 mM Tris-Cl and a pH 7.4). Each sample was mixed with 6X SDS buffer, boiled in water at 95°C for 10 minutes, and then electrophoresed on 15%, 10-well and 0.75 mm SDS-PAGE gels. Then, the ratio of the insoluble fraction to the soluble fraction in the obtained gel was calculated using a GelAnalyzer. The result showed that, for the control MSP1E3D1 protein, as the temperature increased, the soluble fraction decreased and the insoluble fraction increased, whereas the esMSP protein was present in a more soluble form at high temperatures (FIG. 4). Also, the result of analysis using the GelAnalyzer showed that the MSP1E3D1 protein underwent a gradual increase in insoluble fraction and was denatured to about 70% of the total weight thereof, whereas only about 30% of the esMSP protein was denatured even at 80°C. This showed that the esMSP protein having two ends linked to each other was more than twice as stable to heat as the linear MSP protein.

### 1-4. Production of nanoperforator esNP (end-spliced NP) including esMSP

POPC (l-palmitoyl-2-oleoyl-sn-glycero-3-phosphocholine) as a lipid was dissolved in chloroform to prepare a lipid solution having a concentration of 25 mg/ml. Then, 121.616 µl of a 25 mg/ml POPC solution was transferred to a glass tube to adjust the concentration of the lipid to 10 mM when dissolved in 400 ml of an NP buffer (40 mM Tris-Cl, 300 mM NaCl, 0.5 mM EDTA, pH 7.4) supplemented with sodium cholate. Then, nitrogen gas was added thereto and then the solution was allowed to stand under vacuum for at least 4 hours to remove the solvent to thereby obtain a lipid film. The obtained lipid film was hydrated with 0.4 ml of an NP buffer supplemented with sodium cholate and was sonicated at 55°C for 15 minutes to obtain a suspension containing the pulverized lipid film. The obtained suspension was treated with Ca-circularized MSP (having a molecular weight of 32 kDa) as a membrane scaffold protein in a suitable amount to adjust the molar ratio of lipid to protein to 120:1, treated or not treated with 2 mM TCEP based on the total amount of the mixed solution, and then treated with the equal amount of bio-beads (4°C, 4 hours) to produce a nanoperforator (NP) containing the Cfa-circularized MSP protein through a self-assembly process.

### Example 2. Production of nanoperforator eslNP containing end-spliced large membrane scaffold protein

### 2-1. Production of plasmid for production of end-spliced large membrane scaffold protein (eslMSP)

In order to produce an end-spliced large MSP including two MSP1E3D1 proteins linked in a circular form, a plasmid expressing the end-spliced membrane scaffold protein was produced. The MSP1E3D1 sequence was inserted between the MSP1E3D1 gene sequence and 7 histidines. Gene cloning was performed using overlap cloning, the sequences of the primers used in the experiment are set forth in Table 4 below, the structure of the final protein CfaC-linker-MSP1E3D1-mSP1E3D1-HisX7-CfaN (eslMSP) is shown in FIG. 5, and the DNA sequence thereof is represented by SEQ ID NO: 2.

**[Table 4]**

| | | |
|---|---|---|
| Insert | Forward primer | AAGCTGAATACCCAGACCTTCAGTAAACTTCGCGAA CAACTGG |
| | Reverse primer | ATGATGATGATGATGCTGGGTATTCAGCTTTTTAGT ATATTCTTCCAGAG |
| Vector | Forward primer | CATCATCATCATCATCATCACTGCCTGTCTTA |
| | Reverse primer | CTGGGTATTCAGCTTTTTAGTATATTCTTCCAGAG |

### 2-2. Purification of eslMSP

BL21(DE3) competent cells were transformed with each of the produced plasmids through heat shock for protein purification. Colonies grown on a solid medium containing 50 µg/ml of kanamycin were inoculated into a liquid medium (LB broth miller) containing 10 ml of kanamycin, cultured for 16 hours at 37°C in a shaking incubator and secondarily inoculated into 600 ml of the same liquid medium. When *E. coli* grew to an OD₆₀₀ of 0.5 to 0.8, protein expression was performed by treatment with 0.5 mM IPTG. After expression at 37°C and 150 rpm for 3 hours, centrifugation was performed at 6,000 rpm for 10 minutes to remove the medium and extract only cells. The cells were resuspended using tris buffer containing 1% Triton X-100 and 2 mM TCEP, and were then disrupted using sonication. The cell substrate was isolated through centrifugation and treated with Ni-NTA agarose beads. Substances that could not be bound to the beads were removed using a buffer containing each of Triton X-100, sodium cholate, and low-concentration imidazole, and the protein to be purified was collected using highly concentrated imidazole. In addition, the result of treatment of the purified protein with DAPase used to remove the N-terminal His-tag of the protein showed that the control MSP1E3D1 protein was reduced in size due to removal of the His-tag through the cleavage reaction, whereas eslMSP had no reaction with DAPase (FIG. 6). As a result, the end-spliced structure of eslMSP was identified.

### 2-3. Production of nanoperforator eslNP (end-spliced large NP) containing eslMSP

POPC (l-palmitoyl-2-oleoyl-sn-glycero-3-phosphocholine) as a lipid was dissolved in chloroform to prepare a lipid solution having a concentration of 25 mg/ml. Then, 121.616 µl of a 25 mg/ml POPC solution was transferred to a glass tube to adjust the concentration of the lipid to 10 mM when dissolved in 400 ml of an NP buffer (40 mM Tris-Cl, 300 mM NaCl, 0.5 mM EDTA, pH 7.4) supplemented with sodium cholate. Then, nitrogen gas was added thereto and then the solution was allowed to stand under vacuum for at least 4 hours to remove the solvent to thereby obtain a lipid film. The obtained lipid film was hydrated with 0.4 ml of an NP buffer supplemented with sodium cholate and was sonicated at 55°C for 15 minutes to obtain a suspension containing the pulverized lipid film. The obtained suspension was treated with the eslMSP (having a molecular weight of 62 kDa) as a membrane scaffold protein in a suitable amount to adjust the molar ratio of lipid to protein to 480:1, and was then treated (4°C, 4 hours) with bio-beads in an amount equal to the total amount of the mixed solution to produce an end-spliced large nanoperforator (eslNP) through a self-assembly process.

### Example 3. Production of double nanoperforator (Di-NP)

### 3-1. Production of plasmid for production of MSP1E3D1 into which Linker-CfaN and CfaC-linker are inserted

DNA sequence information for CfaN and CfaC was acquired from Stevens A.J., et al., 2016, Design of a Split Intein with Exceptional Protein Splicing Activity, J. Am. Chem. Soc., and the linker sequence used herein was a general GGGGSGGGGSGGGGS linker. Gene synthesis was outsourced (Cosmogene Tech, Korea), and the obtained DNA sequence is shown in Table 5 below. Initial PCR was performed using primers designed such that, in the pET28a plasmid into which MSP1E3D1 was inserted, for the linker-CfaN, 15 bp at the 3' end of the MSP1E3D1 gene excluding the end codon were complementary to 15 bp at the 5' end of the pET28a vector including the end codon behind the same, and for the CfaC-linker, 15 bp at the 5' end of the MSP1E3D1 gene were complementary to 15 bp at the 3' end of the pET28a vector before the same. After PCR was completed, the result was treated with a Dpn1 solution at 37°C for 1 hour in order to prevent self-ligation and PCR products other than DNA were removed. In order to ligate the pET28a to the inserts thus produced, reaction was performed in the presence of an overlap cloner (ELPIS, Korea) at 37°C for 1 hour to induce stable hydrogen bonding between pET28a and the inserts. 7 µl of the DNA solution obtained through the cloning process was put into 100 µl of a competent *E*. *coli* TOP10 solution, incubated on ice for 30 minutes, and then heat-treated at 42°C for 45 seconds. 900 µl of LB (Luria-Bertani) liquid medium was added to the reaction solution, incubated (37°C, 1 hour), and centrifuged (13,000 rpm, 10 minutes) to collect the cells. The collected cell solution (0.1 ml) was smeared and cultured on kanamycin LB solid medium (37°C), and one of the colonies that formed was cultured in 10 ml LB liquid medium containing 0.1% kanamycin for 18 hours (37°C). The culture solution was sonicated and purified to obtain a plasmid having a nucleotide sequence of MSP1E3D1-linker-CfaN (SEQ ID NO: 3) and a plasmid containing a nucleotide sequence of CfaC-linker-MSP1E3D1 (SEQ ID NO: 4) as a result. The sequences of the plasmids were identified by the company (Cosmo Jintech, Korea). The sequences of the primers, detailed compositions, and reaction conditions used in the above process are shown in Table 6.

**[Table 5]**

| | | |
|---|---|---|
| linker-CfaN (pUCIDT vector) | 5'- ggtggaggaggctctggtggaggcggtagcggaggcggagggtcgtgcctg tcttacgacacagagattctgaccgttgaatatggattccttcctatcggt aagatcgtggaggaacggattgaatgcacagtctatacggtagataaaaat ggctttgtgtatacacaacctattgctcagtggcataaccggggagaacag gaagttttcgaatactgcttagaagacggttcgattatccgtgcaacgaaa gatcacaaatttatgacgaccgacggtcagatgttaccgattgatgagatt ttcgaacggggttagacctgaaacaagttgatggtttgccg-3' | |
| | linker | 5'- ggtggaggaggctctggtggaggcggtagcggaggcgga gggtcg-3' |
| | CfaN | 5'- tgcctgtcttacgacacagagattctgaccgttgaatat ggattccttcctatcggtaagatcgtggaggaacggatt gaatgcacagtctatacggtagataaaaatggctttgtg tatacacaacctattgctcagtggcataaccgggagaa caggaagttttcgaatactgcttagaagacggttcgatt atccgtgcaacgaaagatcacaaatttatgacgaccgac ggtcagatgttaccgattgatgagattttcgaacggggg |
| | | ttagacctgaaacaagttgatggtttgccg-3' |
| CfaC-linker (pUCSMART vector) | 5'- atggtcaagatcattagtcgtaagagtctgggcactcaaaacgtctacgat attggagtagaaaaagatcataatttttgctgaagaatggctggtggcc tctaactgcttcaacggtggaggaggctctggtggaggcggtagcggaggc ggagggtcg-3' | |
| | CfaC | 5'- atggtcaagatcattagtcgtaagagtctgggcactcaa aacgtctacgatattggagtagaaaaagatcataatttt ttgctgaagaatgggctggtggcctctaactgcttcaac -3' |
| | Linker | 5'- ggtggaggaggctctggtggaggcggtagcggaggcgga gggtcg-3' |

**[Table 6]**

| | | | | |
|---|---|---|---|---|
| Primer | CfaN-linker-MSP1E3D1 | vector(MS P1E3D1-pET28a, 6400bp) | Forwa rd | 5'- GCTTGCGGCCGCACTCGAGCACCA- 3' |
| | | | Rever se | 5'- CTGGGTATTCAGCTTTTTAGTATAT TCTTCCAGAGCTGACAGAAAACTG- 3' |
| | CfaC-linker-MSP1E3D1 | | Forwa rd | 5'- TAGAAATAATTTTGTATGGTCAAGA TCATTAGTCGTAAGAGTCTGGG-3' |
| | | | Rever se | 5'- CCTTCTTAAAGTTAACGACCCTCCG CCTCC-3' |
| PCR compositi on | Each primer 1 µℓ, vector 3 µℓ, dNTP 4 µℓ, 10X reaction buffer 5 µℓ, HiPi DNA polymerase 0.5 µℓ, and distilled water 35.5 µℓ | | | |
| Vector PCR condition s | Initial denaturation (95°C, 3 minutes) 30-cycle repetition: denaturation (95°C, 5 seconds) → annealing (62°C, 5 seconds) → extension (72°C, 6 minutes) Final extension (72°C, 3 minutes) | | | |
| Insert PCR condition s | Initial denaturation (95°C, 3 minutes) 30-cycle repetition: denaturation (95°C, 3 seconds) → annealing (62°C, 3 seconds) → extension (72°C, 1 minute) Final extension (72°C, 3 minutes) | | | |
| Dpn1 treatment condition s | 5 µℓ (10X reaction buffer4) + 44 µℓ (PCR product of vector or insert) + 1 **µℓ** (Dpn1 solution), at 37°C for 1 hour | | | |
| Overlap cloner ligase treatment | 4 **µℓ** (vector solution) + 4 **µℓ** (insert solution) + 1 **µℓ** (10X reaction buffer) + 1 **µℓ** (overlap cloner solution) | | | |

### 3-2. Purification of two types of proteins bound to Cfa intein domains and identification of covalent bond

Two types of proteins, namely MSP1E3D1-linker-CfaN (MSP-CfaN) and CfaC-linker-MSP1E3D1 (CfaC-MSP), were purified. Specifically, MSP-CfaN was expressed and purified in the same manner as in the example above, except that after desalting, the final result was obtained in a splicing buffer (100 mM sodium phosphate, 150 mM NaCl, 1 mM EDTA, pH 7.2). For CfaC-MSP, the constructed plasmid was transformed into BL21 (DE3) competent cells through heat shock for protein purification. Colonies grown on a solid medium containing 50 µg/ml of kanamycin were inoculated into a liquid medium containing 10 ml of kanamycin (LB broth miller), incubated in a shaking incubator at 37°C, and then secondarily inoculated into 600 ml of the same liquid medium. When *E. coli* grew to an OD₆₀₀ of 0.5 to 0.8, protein expression was performed by treatment with 0.5 mM IPTG. After expression at 16°C and 120 rpm for 16 hours, the result was centrifuged at 6,000 rpm for 10 minutes to remove the medium and thereby extract only cells. The cells were resuspended using tris buffer containing 1% Triton X-100 and 2 mM TCEP, and were then disrupted using sonication. The cell substrate was isolated through centrifugation and treated with Ni-NTA agarose beads. Then, substances that could not be bound to the beads were removed using a buffer containing each of 2M urea-containing Triton X-100, sodium cholate, and low-concentration imidazole, and the protein to be purified was collected using highly concentrated imidazole. CfaC-MSP obtained by isolation from the Ni-NTA agarose beads through highly concentrated imidazole was used after the highly concentrated urea was removed from the protein solution through dialysis using a splicing buffer. In order to determine whether or not the obtained MSP-CfaN and CfaC-MSP form a covalent bond, each of them was diluted at a molar ratio of 1:1.3, treated with 2 mM TCEP, and reacted at 25°C for 15 minutes. Then, the same volume of the protein was reacted at 30°C for 1 hour. As a result, the protein was trans-spliced over time to produce an MSP-MSP conjugate having a high molecular weight (arrow in FIG. 7).

### 3-3. Production of covalently linked double nanoperforator Di-NP

In order to produce a PTS (protein trans-splicing) double nanoperforator (Di-NP), nanoperforators having CfaN and CfaC (NP-CfaN and CfaC-NP, respectively) were separately produced using each of the MSP-CfaN and CfaC-MSP produced above while excluding treatment with TCEP from the process described in Example 1-4. Pure nanoperforators were isolated through size exclusion chromatography from the nanoperforators thus produced in a buffer for splicing (100 mM sodium phosphate, 150 mM NaCl, 1 mM EDTA, pH 7.2). Then, NP-CfaN and CfaC-NP were prepared at a molar ratio of 1:1.3, treated with 2 mM TCEP, and reacted at 25°C for 15 minutes. Then, equal volumes of NP-CfaN and CfaC-NP solutions were mixed and reacted at 33°C for 1 hour to produce Di-NP in which two NPs were covalently linked. It was identified through size exclusion chromatography that the double nanoperforator including two nanoperforators bound to each other had about twice the molecular weight of the single nanoperforator. The double nanoperforator was isolated through size exclusion chromatography.

### Example 4. Production of antibody-binding nanoperforator FcNP

### 4-1. Production of plasmid for production of antibody-binding membrane scaffold protein FcMSP

DNA sequence information for the Fc region-binding peptide (Fcbp) was acquired from Weonu Choe, et al., 2016, Fc-binding Ligands of Immunoglobulin G: An Overview of High Affinity Proteins and Peptides, Materials (Basel), and the linker sequence used herein was a general GGGGSGGGGSGGGGS linker. Gene synthesis was outsourced (Cosmogene Tech, Korea), the obtained DNA sequence is shown in Table 7 below and the schematic diagram of the structure thereof is shown in FIG. 8. Initial PCR was achieved by performing vector linearization in front of the 7XHIS sequence of the pET28a plasmid into which MSP1E3D1 was inserted, and using primers designed such that 15 bp at both 5' and 3' ends of the synthesized insert gene overlapped the vector. After PCR was completed, the result was treated with a Dpn1 solution at 37°C for 1 hour in order to prevent self-ligation and PCR products other than DNA were removed. In order to ligate the pET28a to the inserts thus produced, reaction was performed in the presence of an overlap cloner (ELPIS, Korea) at 37°C for 1 hour to induce stable hydrogen bonding between pET28a and the inserts. 7 µl of the DNA solution obtained through the cloning process was put into 100 µl of a competent *E. coli* TOP10 solution, incubated on ice for 30 minutes, and then heat-treated at 42°C for 45 seconds. 900 µl of LB (Luria-Bertani) liquid medium was added to the reaction solution, incubated (37°C, 1 hour) and centrifuged (13,000 rpm, 10 minutes) to collect the cells. The collected cell solution (0.1 ml) was smeared and cultured on kanamycin LB solid medium (37°C), and one of the colonies that formed was cultured in 10 ml LB liquid medium containing 0.1% kanamycin for 18 hours (37°C). The culture solution was sonicated and purified to obtain a plasmid as a result. The sequence of the plasmid was identified by the company (Cosmo Jintech, Korea). The sequences of the primers used in the above process are shown in Table 7, and detailed compositions and reaction conditions are shown in Table 8.

**[Table 7]**

| | | |
|---|---|---|
| Fcbp-linker (pET28a vector) | 5'- gactgtgcatggcacctgggagaactggtctggtgcaccggcggtggcggta gcggcggtggcggtagcggcggtggcggtagcggt-3' | |
| Insert | Forward primer | 5'- AGGAGATATACCATGGACTGTGCATGGCACC-3' |
| | Reverse primer | 5'- GATGATGATGATGACCGCTACCGCCACCGCC-3' |
| Vector | Forward primer | 5'-CATCATCATCATCATCATCACCGA-3' |
| | Reverse primer | 5'-CATGGTATATCTCCTTCTTAAAGT-3' |

**[Table 8]**

| | |
|---|---|
| PCR compositi on | Each primer 1 **µℓ,** vector 3 **µℓ,** dNTP 4 **µℓ,** 10X reaction buffer 5 **µℓ,** HiPi DNA polymerase 0.5 **µℓ** and distilled water 35.5 **µℓ** |
| Vector PCR condition s | Initial denaturation (95°C, 3 minutes) 30-cycle repetition: denaturation (95°C, 5 seconds) → annealing (62°C, 5 seconds) → extension (72°C, 6 minutes) Final extension (72°C, 3 minutes) |
| Insert PCR condition s | Initial denaturation (95°C, 3 minutes) 30-cycle repetition: denaturation (95°C, 3 seconds) → annealing (62°C, 3 seconds) → extension (72°C, 1 minute) Final extension (72°C, 3 minutes) |
| Dpn1 treatment condition s | 5 **µℓ** (10X reaction buffer4) + 44 **µℓ** (PCR product of vector or insert) + 1 **µℓ** (Dpn1 solution), at 37°C for 1 hour |
| Overlap cloner ligase treatment | 4 **µℓ** (vector solution) + 4 **µℓ** (insert solution) + 1 **µℓ** (10X reaction buffer) + 1 **µℓ** (overlap cloner solution) |

### 4-2. Purification of FcMSP

FcMSP was purified in the same manner as eslMSP of Example 2-2, and was identified through an SDS-PAGE gel. The result showed that MSP including an Fc-binding domain was purified normally (FIG. 9).

### 4-3. Production of nanoperforator FcNP including FcMSP

POPC (l-palmitoyl-2-oleoyl-sn-glycero-3-phosphocholine) as a lipid was dissolved in chloroform to prepare a lipid solution having a concentration of 25 mg/ml. Then, 121.616 µl of a 25 mg/ml POPC solution was transferred to a glass tube to adjust the concentration of the lipid to 10 mM when dissolved in 400 ml of an NP buffer (40 mM Tris-Cl, 300 mM NaCl, 0.5 mM EDTA, pH 7.4), supplemented with sodium cholate. Then, nitrogen gas was added thereto and then the solution was allowed to stand under vacuum for at least 4 hours to remove the solvent to thereby obtain a lipid film. The obtained lipid film was hydrated with 0.4 ml of an NP buffer supplemented with sodium cholate and was sonicated at 55°C for 15 minutes to obtain a suspension containing the pulverized lipid film. The obtained suspension was treated with the Fc-MSP (having a molecular weight of 35.2 kDa) as a membrane scaffold protein in an amount to adjust the molar ratio of lipid to protein to 120. Then, the result was treated with a reducing agent (TCEP) in an amount of 2 mM based on the total volume of the mixed solution. Then, the result was treated with bio-beads in the same amount as the total mixed solution (4°C, 4 hours), to produce a nanoperforator including an FC-binding domain (FcNP) through a self-assembly process.

### Example 5. Production of antibody-binding nanoperforator Fc-eslNP containing end-spliced large membrane scaffold protein

### 5-1. Production of plasmid for production of antibody-binding membrane scaffold protein (Fc-eslMSP)

DNA sequence information for the Fc region-binding peptide (Fcbp) was acquired from Weonu Choe, et al., 2016, Fc-binding Ligands of Immunoglobulin G: An Overview of High Affinity Proteins and Peptides, Materials (Basel), and the linker sequence used herein was a general GGGGS linker. Gene synthesis was outsourced (Cosmogene Tech, Korea), the obtained DNA sequence is shown in Table 9 below, and the schematic diagram of the structure thereof is shown in FIG. 10. For PCR, the second GGGGS sequence was removed from the GGGGSGGGGSGGGGS linker of the pET28a plasmid into which eslMSP was inserted, and a primer capable of inserting Fcbp at the corresponding position was designed. PCR was performed using site-directed mutagenesis. After PCR was completed, the result was treated with a Dpn1 solution at 37°C for 1 hour in order to delete the methylated plasmid. Then, the remaining PCR product was further purified using a PCR purification kit (ELPIS, Korea). 7 µl of the DNA solution obtained through the cloning process was put into 100 µl of a competent *E. coli* TOP10 solution, incubated on ice for 30 minutes, and then heat-treated at 42°C for 45 seconds. 900 µl of LB (Luria-Bertani) liquid medium was added to the reaction solution, followed by incubation (37°C, 1 hour) and centrifugation (13,000 rpm, 10 minutes) to collect the cells. The collected cell solution (0.1 ml) was smeared and cultured on kanamycin LB solid medium (37°C), and one of the colonies that formed was cultured in 10 ml LB liquid medium containing 0.1% kanamycin for 18 hours (37°C). The culture solution was sonicated and purified to obtain a plasmid as a result. The sequence of the plasmid was identified by the company (Cosmo Jintech, Korea). The sequences of the primers used in the above process are shown in Table 9, and the detailed compositions and reaction conditions are shown in Table 10.

**[Table 9]**

| Fcbp (pET28a vector) | 5'-gactgtgcatggcacctgggagaactggtctggtgcacc-3' | |
|---|---|---|
| Insert | Forward primer | 5'- GACTGTGCATGGCACCTGGGAGAACTGGTCTGGTG CACC GGAGGCGGAGGGTCGTCTAC-3' |
| | Reverse primer | 5'- AGAGCCTCCTCCACCGTTGAAGC -3' |

**[Table 10]**

| PCR compositi on | Each primer 2.5 **µℓ,** vector 3 **µℓ,** dNTP 1 **µℓ,** 5X Phusion HF buffer 10 **µℓ,** Phusion polymerase 0.5 **µℓ** and distilled water 30.5 **µℓ** |
|---|---|
| Insert PCR condition s | Initial denaturation (95°C, 3 minutes) 25-cycle repetition: denaturation (95°C, 30 seconds) → annealing (59°C, 30 seconds) → extension (72°C, 3 minutes, 30 seconds) Final extension (72°C, 10 minutes) |
| Dpn1 treatment condition s | 5.5 **µℓ** (10X reaction buffer 4) + 50 **µℓ** (PCR product of vector or insert) + 2 **µℓ** (Dpn1 solution), at 37°C for 1 hour |

### 5-2. Purification of Fc-eslMSP

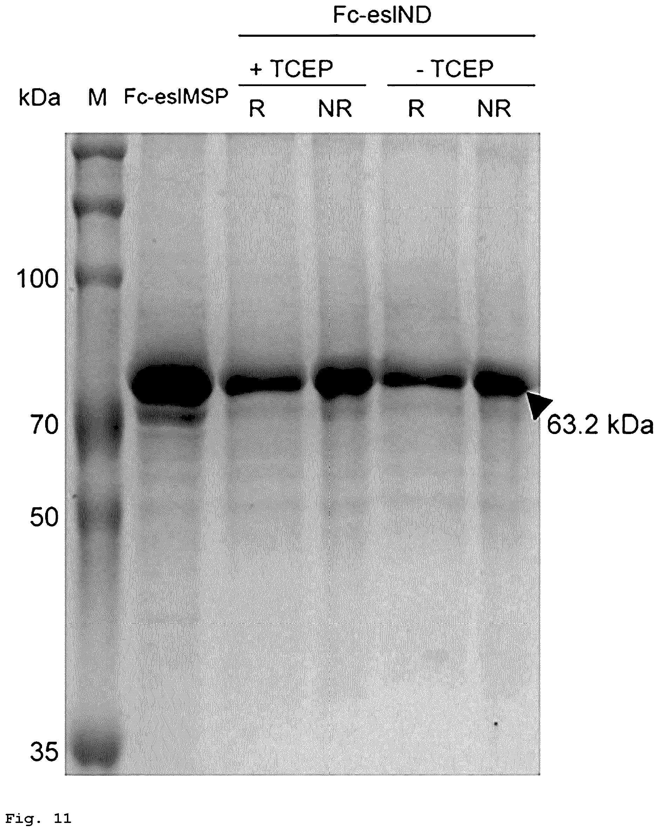

Fc-eslMSP was purified in the same manner as the eslMSP of Example 2-2, and was identified through an SDS-PAGE gel. The result showed that Fc-eslMSP including an Fc-binding domain was purified normally (FIG. 11).

### 5-3. Production of nanoperforator Fc-eslNP including Fc-eslMSP

POPC (1-palmitoyl-2-oleoyl-sn-glycero-3-phosphocholine) as a lipid was dissolved in chloroform to prepare a lipid solution having a concentration of 25 mg/ml. Then, 121.616 µl of a 25 mg/ml POPC solution to adjust the concentration of the lipid to 10 mM when dissolved in 400 ml of an ND buffer (40 mM Tris-Cl, 300 mM NaCl, 0.5 mM EDTA, pH 7.4), supplemented with sodium cholate, was transferred to a glass tube. Then, nitrogen gas was added thereto and then the solution was allowed to stand under vacuum for at least 4 hours to remove the solvent to thereby obtain a lipid film. The obtained lipid film was hydrated with 0.4 ml of an ND buffer supplemented with sodium cholate and was sonicated at 55°C for 15 minutes to obtain a suspension containing the pulverized lipid film. The obtained suspension was treated with the Fc-eslMSP (having a molecular weight of 63.2 kDa) as a membrane scaffold protein in a suitable amount to adjust the molar ratio of lipid to protein to 480:1. Then, the result was treated with a reducing agent (TCEP) in an amount of 2 mM based on the total volume of the mixed solution. Then, the result was treated with bio-beads in the same amount as the total mixed solution (4°C, 4 hours), to produce a nanoperforator including an FC-binding domain (Fc-eslND) through a self-assembly process.

### Example 6. Production of PEG-bound nanoperforators

To improve the stability of the construct, BM(PEG)3 (1,11-bismaleimido-triethyleneglycol) from the Thermo Fisher Scientific was linked to the eslNP and Fc-eslNP produced in Examples 2-3 and 5-3. Specifically, the produced eslNP and Fc-eslNP were treated with 5 mM TCEP at room temperature for 30 minutes, and then the TCEP was removed through desalting or concentration. The residue was treated with BM(PEG)2 or BM(PEG)3 in a molar amount double that of the membrane protein of each of eslNP and Fc-eslNP, followed by reaction at room temperature for 1 hour. Then, in order to terminate the reaction, the reaction solution was treated with 10 mM DTT at room temperature for 15 minutes to produce PEG-eslND and PEGylated Fc-eslND in which PEG was bound to eslNP and Fc-eslNP, respectively.

### Experimental Example 1. Identification of structure of nanoperforators

### 1-1. Determination of structure of esND

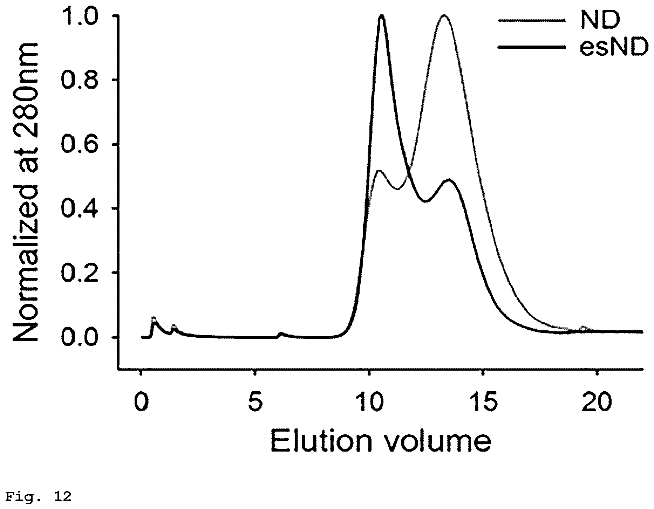

The size and shape of the esND produced in Example 1-4 were identified through size exclusion chromatography (SEC), dynamic light scattering (DLS), and electron microscopy. Specifically, the produced esNP was purified through size exclusion chromatography (SEC). When esND was produced without adding TCEP, two main peaks of ND and esND were observed (FIG. 12).

### 1-2. Determination of structure of eslND

The eslND produced in Example 2-3 was identified through size exclusion chromatography (SEC). As a result, a shift of the peak due to the increased molecular weight compared to the ND produced with MSP1E3D1 was observed (left side of FIG. 13). In addition, the diameter of each nanoperforator was measured through dynamic light scattering (DLS) analysis. The result showed that the ND had a diameter of 12.37 nm, whereas the eslND had a diameter of about twice that (25.70 nm) (right side of FIG. 13), which was consistent with the expected values upon protein design. In addition, the schematic structure of the large nanoperforator is shown at the bottom of FIG. 13, and the green part represents a region linked via a covalent bond based on intein trans-splicing.

### 1-3. Determination of structure of Di-ND

It was found through size exclusion chromatography that the Di-ND produced in Example 3-3 had a molecular weight about twice that of each nanoperforator (FIG. 14A) and it was found through SDS-PAGE that the Di-ND thus separated had a molecular weight corresponding to a total of two MSP1E3D1 proteins (FIG. 14B). In addition, the result of dynamic light scattering analysis showed that the conventional nanoperforator had a diameter of about 12 nm, whereas Di-ND had a diameter of about 20 nm (FIG. 14C). Taking into consideration the fact that in a dynamic light scattering method, an average of a length of a long axis and a length of a short axis is measured to determine the diameter of an oval structure, it can be clearly seen that Di-ND had a structure in which two nanoperforators are linked.

### 1-4. Identification of structure of FcND

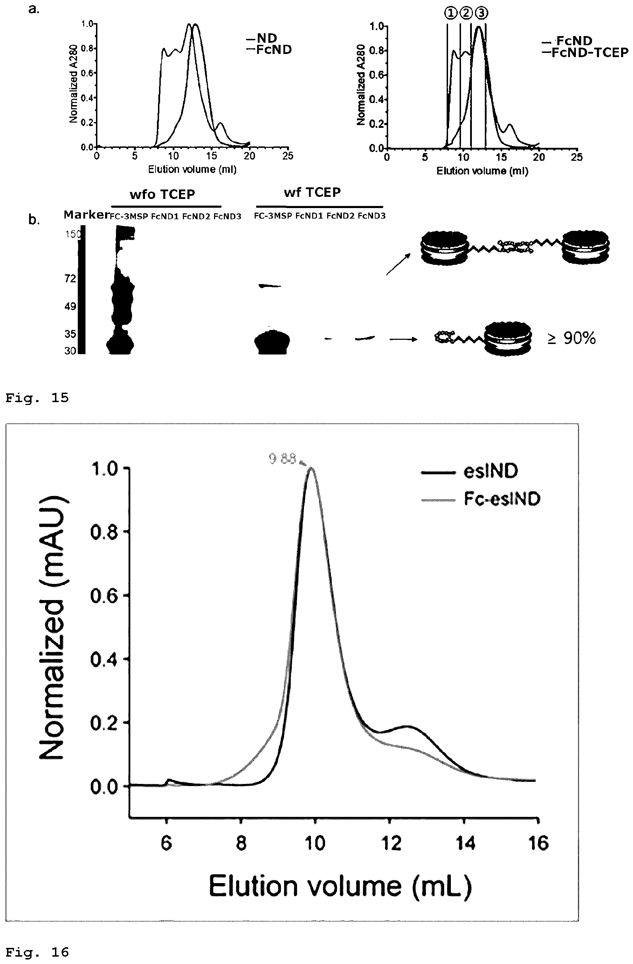

The FcND produced in Example 4-3 was purified through size exclusion chromatography (SEC). It was found that by adding TCEP, a reducing agent, during the purification process, FcND in aggregate and dimer forms observed in elution volumes of about 8.5 ml and 10.5 ml was reduced, and that the yield of monocyclic FcND capable of binding to the Fc region of the antibody increased (FIG. 15A). Similar to the result of SEC, the result of SDS-PAGE analysis performed on three elution volumes also showed that the yield of monocyclic FcND was increased by treatment with TCEP (FIG. 15B). Here, in FIG. 15B, the red part schematically illustrates a linker structure and the yellow part represents an Fc-binding domain.

### 1-5. Identification of structure of Fc-eslND

The Fc-eslND produced in Example 5-3 was compared with eslND using size exclusion chromatography. The result showed that the Fc-eslND had a molecular weight similar thereto (FIG. 16). In addition, the peak of 9.88 mL obtained through the above process was analyzed through SDS-PAGE (FIG. 11). As a result, the same molecular weight as Fc-eslMSP was observed, indicating that Fc-eslMSP was formed.

### 1-6. Comparison in structure between esNP and esdNP produced with and without TCEP

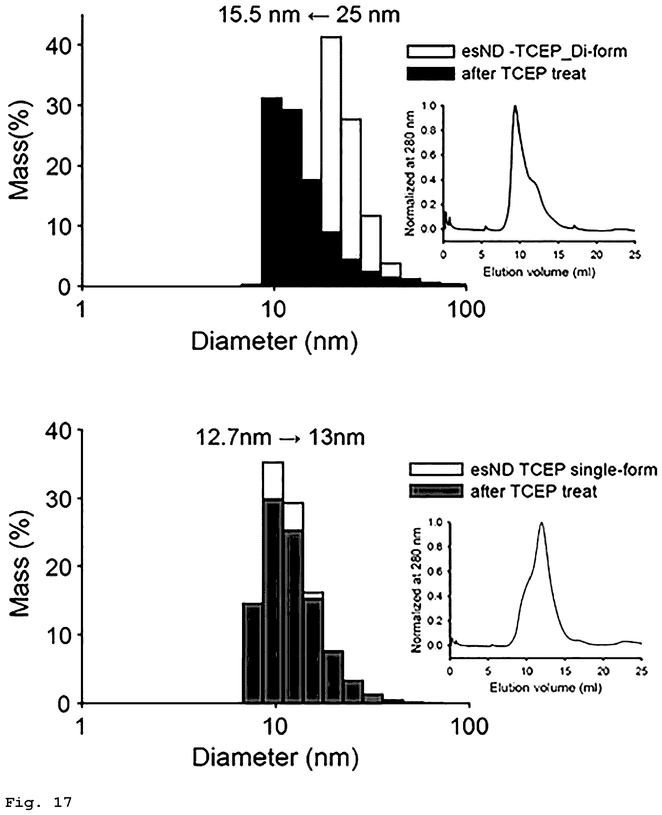

essND, which was an esNP produced with esMSP purified through addition with TCEP, and esdNP (including esNPs linked by a disulfide bond), which was an esNP produced with esMSP purified without addition with TCEP, as obtained in Example 1-2 above, were purified through size exclusion chromatography (SEC). When esNP was produced without addition with TCEP, two main peaks were observed. At this time, the first peak was identified as esdNP and the subsequent peak was identified as esNP (FIG. 17). The peaks were measured by dynamic light scattering (DLS). As a result, the size of esdNP was 25 nm, which was about twice that of esNP (about 12 nm). The esdNP linked by a disulfide bond decreased in size because the disulfide bond was broken when DLS was measured again after treatment with TCEP, so the structure of esdNP could be determined more accurately (FIG. 17). In addition, the esNP could be obtained by treatment with TCEP in the nanodisc production process, and DSL confirmed that the obtained esNP did not change in size even after treatment with TCEP, which indicates that two types of NPs can be obtained depending on whether or not treatment with TCEP is performed (FIG. 18).

### Experimental Example 2. Determination of antiviral effect of nanoperforator

### 2-1. esNP

In order to determine the antiviral effect of NPTG and esdNPTG (NP and esdNP containing TG as a receptor) respectively produced by changing the lipid to a mixture of 70% POPC and 30% TG, instead of 100% POPC, during lipid film production at the beginning of the NP and esdNP production process, MDCK cells were infected with influenza virus A/PR/8/34 strain, then treated with NPTG or esdNPTG, and the decrease in cytopathic effect due to inhibition of viral infection was measured. Specifically, 200 µl of 3 × 10⁵ cells/ml MDCK cells were seeded into each well of a 96-well cell culture plate and cultured in a 5% CO₂ incubator at 37°C for 24 hours. Then, the medium was removed from the cells, and 100 µl of PBS was dispensed into each well, removed and washed twice. The influenza virus was diluted to an MOI (multiplicity of infection) of 0.01 and mixed with various concentrations of esNP (2× dilution from 1 µM). Then, 100 µl of a 3 × 10⁵ pfu/ml influenza virus was prepared and seeded into each well to induce infection at 37°C for 1 hour. Then, the result was reacted for 1 hour in a 5% CO₂ incubator at 37°C, and then PBS-washed cells were treated therewith. The cells were cultured in a 5% CO₂ incubator at 37°C for 18 hours, the medium was removed therefrom, and 100 µl of 4% formaldehyde was dispensed into each well and reacted at 25°C for 1 hour to induce immobilization. After immobilization, 100 µl of a 0.5% crystal violet solution was dispensed into each well to perform staining at 25°C for 1 hour. Then, the crystal violet solution was removed, the dried crystal violet was dissolved by treatment of each well with 100 µl of methanol, and absorbance at a wavelength of 570 nm was measured with a spectrophotometer to determine the effect of reducing cell lesions. At this time, a group in which only virus and MDCK cells were cultured without an antiviral agent was set as a negative control, and a group in which only MDCK cells were cultured without viral infection was set as a positive control, and NP produced with the conventional MSP1E3D1 that was not end-spliced was set as a control. The effect of the antiviral agent on reduction of the cytopathic effect was measured. In addition, the concentration of the treated sample was based on the protein concentration measured by DC assay (detergent-compatible protein concentration determination), and thus the antiviral effect of the same molar amounts of NDTG and esdNPTG was determined.

The result of analysis of CPE inhibition by esdNPTG having an end-spliced form produced in Example 1-4 showed that esdNPTG exhibited increased CPE inhibition compared to NPTG, as shown at the top of FIG. 19, and an improvement of about 70% in antiviral activity at the highest concentration (1 µM) compared to the conventional NPTG.

### 2-2. Di-NP

The double nanoperforator (including two nanoperforators linked through a covalent bond) produced in Example above was subjected to CPE inhibition assay in the same manner as in Experimental Example 2-1, and the result is shown in a graph. Here, Di-NPTG was compared with the conventional NPTG. In addition, the concentration of the treated sample was based on the protein concentration measured by DC assay (detergent-compatible protein concentration determination). The nanoperforator had a form in which two nanoperforators are linked, so the calculated number of moles was 1/2 of the original amount.

The result showed that the Di-NPTG exhibited an at least 2.5-fold increase in antiviral efficacy at the highest concentration compared to NPTG (bottom of FIG. 19). In addition, when comparing the effect of reducing cell lesions through an optical microscope, with the passage of time after infection with the PR8 influenza virus, in NPTG, dead MDCK cells floated, like the negative control group, even at the highest concentration, and voids were formed (FIG. 20A). However, Di-NPTG exhibited relatively little antiviral efficacy at a low concentration, but most of viable cells were normally and well adhered at the maximum concentration, like the positive control group, even 48 hours after infection (FIG. 20B).

### 2-3. eslNP

eslNP having a diameter of about 25 nm produced in Example 2-3 was subjected to CPE inhibition assay in the same manner as in Experimental Example 2-1. Here, an NP having a diameter of 13 nm was used as a control group.

The result showed that the control group, NDTG exhibited an inhibition up to about 20% of viral activity, whereas eslNDTG exhibited an improved inhibition of 80%, and ND and eslND, containing no receptor, did not exhibit any effect (FIG. 21).

### 2-4. FcNP

The FcNP produced in Example 4-3 above was subjected to microneutralization assay, and the result is shown as a graph. Specifically, first, NP or FcNP was diluted 2×, added at 50 µl/well to a 96-well plate, and reacted at 37°C for 1 hour. Then, 50 µl of A/PR/8/34 virus was added at a concentration of MOI (multiplicity of infection) of 0.01, followed by reaction at 37°C for 1 hour. Then, 100 µl of MDCK cells were added at a concentration of 2×10⁵ cells/ml to each well, followed by reaction at 37°C in a 5% CO₂ humidified incubator for 18 hours. 50 µl of fluorescently labeled 4-methylumbelliferyl-N-acetyl-α-D-neuraminic acid was added at a concentration of 500 µM to each well, followed by reaction at 37°C for 1 hour. Then, fluorescence measurements were measured using a fluorescence plate reader (excitation 355 nm/ emission 460 nm). In addition, the concentration of the treated sample was based on the protein concentration measured through a DC assay (detergent-compatible protein concentration determination), which means that the antiviral effect of the same molar amounts of NP and FcNP was analyzed.

The result showed that FcNP exhibited improved antiviral efficacy compared to NP and increased virus-neutralizing efficacy compared to the case of treatment only with the antibody (FIG. 22).

### 2-5. Fc-eslNP

The Fc-eslNP produced in Example 5-3 was subjected to a plaque reduction assay, and the result is shown as a graph. Specifically, first, an antivirus-neutralizing antibody (Abs) or a mixture of Abs and Fc-eslNP was diluted 5x and reacted with 1 mL of A/PR/8/34 virus having a concentration of 100 PFU at 37°C for 1 hour. Then, the reaction solution was added at 1 mL/well to MDCK cells cultured to about 90% confluence in a 6-well plate and reacted at 37°C for 1 hour. The reaction solution was washed with PBS and treated with 2 mL of a medium containing DMEM, 1% agar, and 2 ug/mL of TPCK-trypsin. Then, the reaction solution was reacted in a humidified incubator at 37°C and 5% CO₂ for 72 hours, 1 mL of 3.65% formaldehyde was added to each well to immobilize the MDCK cells, and the cells were stained with 0.5% crystal violet to detect plaques. The result showed that antiviral efficacy was improved when a mixture of Fc-eslNP and the corresponding antibody was used, compared to when the antivirus-neutralizing antibody was used alone. At this time, Fc-eslNP_{fusion}, which was an eslNP produced using the fusion composition (POPC 55%, DOPS 15% and cholesterol 30%) as a lipid, exhibited better activity than Fc-eslNP_{popc}, which was an Fc-eslNP produced using POPC (1-palmitoyl-2-oleoyl-sn-glycero-3-phosphocholine) as a lipid (FIG. 23). This indicates that the Fc-eslNP_{fusion} construct bound to the antivirus-neutralizing antibody can perforate the virus in the cells.

### Experimental Example 3. Determination of stability of nanoperforator

### 3-1. Determination of thermal durability and aggregation resistance of esdNP

300 µl of 100 µM esdND produced in Example 1-4 was incubated at 37°C for 24 hours, and then a change in void peaks was observed through size exclusion chromatography (SEC). Here, ND was used as a control group.

As a result, the esdND had almost no SEC change, whereas the ND exhibited an increase in void peak, which indicates that the esdND had higher resistance to temperature than the control group ND (FIG. 24).

### 3-2. Determination of stability of PEG-bound eslND

In order to determine the stability and degrading enzyme resistance of PEG-eslND produced by attaching PEG to the eslND in Example 6, 0.4 mg/ml of PEGylated nanodiscs were treated with 100 nM trypsin, and whether or not structural degradation occurred was observed and compared at 0, 1, 2, 3, 4, 24, 48, 72, and 96 hours while reaction proceeded at 37°C.

The result showed that the degradation of the structure by trypsin was clearly reduced in the structure bound with PEG. In the conventional eslND, upon exposure to 100 nM trypsin for 24 hours or more, all proteins were degraded, whereas in PEG-eslND, at least 50% of the proteins remained non-degraded up to 96 hours (FIG. 25). Considering that proteins that do not form a nanodisc structure are known to be completely degraded by trypsin within a short time, this phenomenon is considered to mean that the nanodisc construct itself has acquired resistance to degradation by trypsin.

### 3-3. Identification of stability distribution pattern and increase in vivo using nanoperforators into which fluorescent dyes are inserted

ND, esdND, eslND and PEG-eslND, which are fluorescent, were produced by mixing 0.5 mol% of DiR fluorescent dye as a lipid component in the conventional method of producing nanoperforators (ND) and the methods of Examples 1-4, 2-3 and 6, respectively. Then, 100 µl of each nanoperforator having a predetermined structure was diluted at a concentration of 5 mg/kg in PBS and injected into mice by intraperitoneal injection (n=3). Immediately after injection, the fluorescence values measured on the surface of each mouse after anesthesia on the 3^{rd} and 6^{th} days were imaged using an IVIS imaging system (FIG. 26A). It was found that continuous fluorescence values were observed in the abdomen due to alfalfa hay contained in the feed in all experimental groups. However, when comparing the fluorescence values in the chest, as a control group, the PBS-injected group had no change in the fluorescence value in the chest immediately after injection, 72 hours after injection, or even after 144 hours, whereas the nanoperforator-injected group exhibited fluorescence in the chest 72 hours after injection. The fluorescence values of the chest were measured and compared (FIG. 26B). 144 hours after injection, the ND experimental group and the eslND experimental group were found to have fluorescence values decreased to 75% and 50%, respectively, compared to 72 hours after injection. On the other hand, it was found that the esdND experimental group and the PEG-eslND experimental group had almost no decrease in fluorescence values. That is, ND and eslND exhibited similar *in-vivo* stability, but esdND maintained remarkably increased fluorescence compared to ND even after 144 hours, and this is considered to be due to increased stability compared to ND due to the cyclic structure of esdND. In addition, *in-vivo* stability due to pegylation was determined. The result showed that when the fluorescence values at 72 hours and 144 hours were compared, PEG-eslND exhibited no decrease in fluorescence value in the chest, similar to esdND and in contrast to eslND, and that eslND exhibited improved in-*vivo* stability due to pegylation. As such, the eslND had a cyclic structure like esdND, but a larger diameter than ND, and thus exhibited lower *in-vivo* stability than esdND. This *in-vivo* stability can be improved by pegylation (PEG-eslND).

### 3-4. Identification of thermal endurance of Fc-eslND

360 µl of FcND produced in Example 4-3 and Fc-eslND produced in Example 5-3 were prepared at a concentration of 0.5 mg/ml. Then, whether or not structural degradation occurred was determined and compared using SDS-PAGE at 0, 1, 2, 4, 8, 12, 18, 24, 36, 48, 60 and 72 hours while reaction proceeded at 37°C.

As a result, there was no significant difference therebetween up to 60 hours, but FcND was completely degraded and disappeared at 72 hours. On the other hand, it was found that more than 60% of Fc-eslND remained (FIG. 27A), which indicates that thermal durability was imparted through circularization of the nanodisc.

### 3-5. Identification of stability of PEG-bound Fc-eslND

In order to determine the stability and degrading enzyme resistance of PEGylated Fc-eslND produced by binding PEG to the Fc-eslND in Example 6, 0.4 mg/ml of PEGylated Fc-eslND was treated with 100 nM trypsin, and then whether or not the structure degradation occurred was determined and compared at 0, 2, 4, 8, 16, 24, and 48 hours while the reaction proceeded at 37°C.

As a result, 50% or more of the conventional FcND was degraded upon exposure to 100 nM trypsin for 3 hours or more, whereas 50% or more of the PEGylated Fc-eslND was degraded upon exposure thereto for 30 hours or more (FIG. 27B), which indicates that degradation of the structure by trypsin was clearly reduced when the PEG was bound thereto. Considering that proteins that do not form a nanodisc structure are known to be degraded by trypsin within a short time, this phenomenon is considered to be due to the fact that the nanodisc construct itself has acquired resistance to degradation by trypsin. Furthermore, it could be concluded that the PEGylated Fc-eslND, the PEG-bound construct, exhibited better ability to withstand trypsin than the non-PEGylated FcND.

### Experimental Example 4. Analysis of antibody-binding patterns of FcND and Fc-eslND

### 4-1. Analysis of antibody-binding pattern in FcND

Fcbp (Fc region binding peptide) can bind to the Fc portion of an antibody only when a pair of cysteine amino acids present in the sequence form a disulfide bond to form a cyclic structure (FIG. 28), so the binding of the nanoperforator to the antibody was measured using surface plasmin resonance (SPR) in order to determine whether or not the binding affinity of the Fcbp introduced with FcND to the antibody was maintained. Specifically, 1 µM of an antibody was immobilized on the surface of a CM5 gold chip, and only the antibody was exposed by blocking with 1 M ethanolamine. Then, ND and FcND were allowed to flow onto the chip surface at 7 µl/min, and the change in the total reflection angle of the gold particles at which the antibody was finely shaken was measured.

The result showed that FcND had a structure capable of binding to an antibody and thus exhibited higher binding affinity thereto than ND (FIG. 29).

### 4-2. Analysis of antibody-binding pattern in Fc-eslND

In order to determine whether or not the Fcbp introduced into Fc-eslND maintained binding affinity to an antibody, a dot blot assay was performed. Specifically, as shown in FIG. 30A, dots were created on a nitrocellulose membrane (NC membrane) with 3 µl of 5 µg/ml A/PR/8/34 virus and then dried at room temperature for 1 hour. Then, the NC membrane was blocked in a 5% solution of skim milk powder in TBST buffer for 1 hour (w/v). A mouse-derived primary antibody against A/PR/8/34 virus was dissolved at a concentration of 1 µg/ml in TBST, and then the NC membrane was treated therewith for 1 hour. The membrane was washed with TBST for 10 minutes, and this was repeated 3 times. Then, a secondary antibody capable of capturing the mouse-derived primary antibody and causing chemiluminescence was dissolved in TBST buffer at a ratio of 1:1000, and then the NC membrane was treated therewith for 1 hour. Then, the result was washed three times with TBST for 10 minutes. Then, a chemiluminescence reaction was induced using ECL, and the film was photosensitized and fixed.

As a result, Fc-eslND had a structure capable of binding to an antibody and thus competitively inhibited the binding of the secondary antibody capable of chemiluminescence to the primary antibody, so the film was not photosensitized. On the other hand, for an eslND having the same size, the film was photosensitized, like the control group (FIG. 30B). Then, it can be seen that the Fcbp introduced into Fc-eslND maintained binding affinity to the antibody.

### Experimental example 5.Identification of in-vivo viral infection inhibition activity of nanoperforator

### 5-1. Improved in-vivo viral infection inhibition activity of esdNP

A lipid film was produced using a mixture of 70% POPC and 30% GD1a receptor, and the inhibitory activity against PR8 virus of NDG (ND including a GD1a receptor) and esdNDG (esdND including a GD1a receptor) produced using the method of Example 1-4 was determined in a mouse model. Specifically, 100 µL of anesthetic (alfaxan:xylazine:PBS=2:1:2) was IP-injected into 6-week-old female Balb/c mice acclimatized to the laboratory environment for one week, followed by challenge infection with a PR8 virus having MLD50 of 4. One hour after challenge infection, NPG or esdNPG was initially administered through IP at a dose of 0.5 mg/kg (n=10), and then was administered two more times at 2-day intervals. Changes in body weight and survival rates over 13 days were observed, recorded, and compared. The result showed that the conventional NPG exhibited a 10% survival rate, whereas the esdNPG exhibited a 3-fold higher survival rate at the same concentration, which means that it is possible to increase antiviral efficacy through structural improvement (FIG. 31).

### 5-2. Improved in-vivo viral infection inhibition of PEGylated eslNP

The inhibitory activity of PEGylated eslNP against PR8 virus infection was tested in a mouse model in the same manner as in Experimental Example 5-1. For this purpose, NPG and PEGylated eslNPG (hereinafter referred to as "PEG-eslNPG") were administered 3 times at 2.5 mg/kg or 0.5 mg/kg over 4 days, and then changes in survival rate and body weight were observed. As a result, unlike the NDG group, all of the infected mice in the PEG-eslNPG-administered group survived at both concentrations, and the change in body weight was also insignificant, at a level similar to that of the uninfected group (FIG. 32). As can be seen from the trypsin resistance test, it is inferred that PEG-eslNPG has superior stability to *in-vivo* protein restriction enzymes. In addition, in comparison with the result of the esdNPG experiment of Experimental Example 5-1, esdNPG not having improved stability to restriction enzymes exhibited a survival rate of only 30% *in vivo,* whereas the PEG-eslNPG exhibited a 100% survival rate, which means that the *in-vivo* stability as well as the increase in the area of the perforator are very important for the efficacy thereof.

### 5-3. Improved in-vivo viral infection inhibitiony activity of PEGylated Fc-eslNP

The inhibitory activity of PEGylated Fc-eslNP (pFc-eslNP) on PR8 virus infection was tested in a mouse model in the same manner as in Experimental Example 5-1. For this purpose, 5 mg/kg (mpk), 2.5 mpk, or 0.5 mpk of Abs (a neutralizing antibody) alone, or a mixture of the same concentration of Abs and 10 mpk, 5 mpk, or 1 mpk of PEGylated Fc-eslNP, was administered intravenously once, and changes in survival rate and weight were observed. As a result, the group administered with Abs 2.5 mpk alone exhibited a survival rate of 40%, whereas the group administered with a mixture of Abs 2.5 mpk and pFc-eslNP 5 mpk exhibited a survival rate of 100% (FIG. 33), which indicates that that the pFc-eslNP is capable of improving the antiviral activity of the neutralizing antibody.

## Claims

1. A plasmid comprising a nucleotide sequence encoding a fusion protein in which an N-terminal domain of an intein is fused to a C-terminus of a membrane scaffold protein and a C-terminal domain of the intein is fused to an N-terminus of the membrane scaffold protein.

2. The plasmid according to claim 1, comprising a nucleotide sequence encoding a fusion protein including two or more repeating membrane scaffold proteins.

3. A plasmid comprising a nucleotide sequence encoding a fusion protein in which a C-terminal domain of an intein is fused to an N-terminus of a membrane scaffold protein.

4. A plasmid comprising a nucleotide sequence encoding a fusion protein in which an N-terminal domain of an intein is fused to a C-terminus of a membrane scaffold protein.

5. A plasmid comprising a nucleotide sequence encoding a fusion protein of an Fc region-binding peptide (Fcbp) and a membrane scaffold protein.

6. The plasmid according to claim 5, wherein an N-terminal domain of an intein is fused to a C-terminus of the fusion protein and a C-terminal domain of the intein is fused to an N-terminus of the fusion protein.

7. A plasmid comprising a nucleotide sequence encoding a fusion protein of an Fc region-binding peptide and two or more repeating membrane scaffold proteins,
wherein an N-terminal domain of an intein is fused to a C-terminus of the fusion protein and a C-terminal domain of the intein is fused to an N-terminus of the fusion protein.

8. A plasmid comprising a nucleotide sequence encoding a fusion protein of an Fc region-binding peptide and a membrane scaffold protein,
wherein a C-terminal domain of an intein is fused to an N-terminus of the fusion protein.

9. A plasmid comprising a nucleotide sequence encoding a fusion protein of an Fc region-binding peptide and a membrane scaffold protein,
wherein an N-terminal domain of an intein is fused to a C-terminus of the fusion protein.

10. An end-spliced membrane scaffold protein (esMSP) having an N-terminus and a C-terminus that are joined together,
the end-spliced membrane scaffold protein (esMSP) produced by expressing the plasmid according to claim 1 and performing purification.

11. The end-spliced membrane scaffold protein (esMSP) according to claim 10, wherein the end-spliced membrane scaffold protein (esMSP) is produced by expressing the plasmid according to claim 1 and performing protein purification by treatment with a reducing agent.

12. An end-spliced double membrane scaffold protein (esdMSP) including two membrane scaffold proteins that are linked through a disulfide bond.

13. The end-spliced double membrane scaffold protein (esdMSP) according to claim 12, wherein the end-spliced double membrane scaffold protein (esdMSP) is produced by expressing the plasmid according to claim 1 and performing purification without treatment with a reducing agent.

14. An end-spliced large membrane scaffold protein (eslMSP) produced by expressing the plasmid according to claim 2 and performing purification.

15. A fusion protein in which a C-terminal domain of an intein is fused to an N-terminus of a membrane scaffold protein,
the fusion protein produced by expressing the plasmid according to claim 3 and performing purification.

16. A fusion protein in which an N-terminal domain of an intein is fused to a C-terminus of a membrane scaffold protein,
the fusion protein produced by expressing the plasmid according to claim 4 and performing purification.

17. An antibody-binding membrane scaffold protein (FcBP-MSP) produced by expressing the plasmid according to claim 5 and performing purification.

18. The antibody-binding membrane scaffold protein (FcBP-MSP) according to claim 17, wherein an N-terminal domain of an intein is fused to a C-terminus of a fusion protein of an Fc region-binding peptide and a membrane scaffold protein, and a C-terminus of the intein is fused to an N-terminus of the fusion protein.

19. The antibody-binding membrane scaffold protein (FcBP-MSP) according to claim 17, produced by performing purification by treatment with a reducing agent.

20. An antibody-binding end-spliced double membrane scaffold protein (FcBP-esdMSP) including two antibody-binding end-spliced membrane scaffold proteins that are linked through a disulfide bond,
the antibody-binding end-spliced double membrane scaffold protein (FcBP-esdMSP) produced by expressing the plasmid according to claim 5 and performing purification.

21. An antibody-binding end-spliced large membrane scaffold protein (FcBP-eslMSP) encoded by the plasmid according to claim 7.

22. A fusion protein of an Fc region-binding peptide and a membrane scaffold protein, encoded by the plasmid according to claim 8,
wherein a C-terminal domain of an intein is fused to an N-terminus of the fusion protein.

23. A fusion protein of an Fc region-binding peptide and a membrane scaffold protein, encoded by the plasmid according to claim 9,
wherein an N-terminal domain of an intein is fused to a C-terminus of the fusion protein.

24. A nanoperforator comprising the end-spliced membrane scaffold protein according to claim 10.

25. A double nanoperforator comprising the end-spliced double membrane scaffold protein according to claim 12.

26. A nanoperforator comprising the end-spliced large membrane scaffold protein according to claim 14.

27. A double nanoperforator wherein the fusion protein according to claim 15, in which the C-terminal domain of the intein is fused to the N-terminus of the membrane scaffold protein, is linked through a covalent bond to the fusion protein according to claim 16, in which the N-terminal domain of the intein is fused to the C-terminus of the membrane scaffold protein.

28. A nanoperforator comprising the antibody-binding membrane scaffold protein (FcBP-MSP) according to claim 17.

29. A double nanoperforator comprising the antibody-binding end-spliced double membrane scaffold protein (FcBP-esdMSP) according to claim 20.

30. A nanoperforator comprising the antibody-binding end-spliced large membrane scaffold protein (Fc-eslMSP) according to claim 21.

31. A double nanoperforator in which a nanoperforator comprising the fusion protein according to claim 22 is linked through a covalent bond to a nanoperforator comprising the fusion protein according to claim 23.

32. A pharmaceutical composition for preventing or treating a viral infection comprising the nanoperforator of any one of claims 24 to 31.

33. The pharmaceutical composition according to claim 32, wherein the nanoperforator further comprises a receptor for a surface antigen of a virus.

34. A method of producing a nanoperforator, the method comprising:
expressing the plasmid according to claim 1 or 5;
purifying an end-spliced membrane scaffold protein (esMSP) or an antibody-binding membrane scaffold protein (FcBP-MSP) by treatment with a reducing agent; and
producing a nanoperforator using a self-assembly process.

35. A method of producing a double nanoperforator, the method comprising:
expressing the plasmid according to claim 1 or 5;
purifying an end-spliced double membrane scaffold protein (esdMSP) or an antibody-binding end-spliced double membrane scaffold protein (FcBP-esdMSP) without treatment with a reducing agent; and
producing a nanoperforator using a self-assembly process.

36. A method of producing a double nanoperforator, the method comprising:
expressing the plasmid according to claim 3 or the plasmid according to claim 8 and performing purification to obtain a fusion protein;
producing a first nanoperforator using a self-assembly process;
expressing the plasmid according to claim 4 or the plasmid according to claim 9 and performing purification to obtain a fusion protein;
producing a second nanoperforator using a self-assembly process; and
covalently bonding the first nanoperforator to the second nanoperforator.
